Europäisches Patentamt

European Patent Office

Office européen des brevets

(19)

(11) **EP 0 665 856 B1**

(12) **EUROPÄISCHE PATENTSCHRIFT**

(45) Veröffentlichungstag und Bekanntmachung des Hinweises auf die Patenterteilung:
**13.01.1999 Patentblatt 1999/02**

(21) Anmeldenummer: **93920853.4**

(22) Anmeldetag: **01.10.1993**

(51) Int Cl.[6]: **C08G 18/48**, C08G 18/50, C08G 18/08, A61L 15/26, A61L 15/60, A61L 15/42, A61L 25/00, C08J 9/00

(86) Internationale Anmeldenummer:
**PCT/EP93/02686**

(87) Internationale Veröffentlichungsnummer:
**WO 94/07935 (14.04.1994 Gazette 1994/09)**

(54) **HYDROPHILE POLYURETHANGELSCHÄUME, INSBESONDERE ZUR BEHANDLUNG VON TIEFEN WUNDEN, WUNDVERBÄNDE AUF BASIS HYDROPHILER POLYURETHANGELSCHÄUME UND VERFAHREN ZUR HERSTELLUNG**

HYDROPHILIC POLYURETHANE GEL FOAMS, PARTICULARLY FOR TREATING DEEP WOUNDS, WOUND DRESSING BASED ON HYDROPHILIC POLYURETHANE GEL FOAMS AND METHOD OF MANUFACTURE

GELS MOUSSANTS HYDROPHILES DE POLYURETHANE, NOTAMMENT POUR LE TRAITEMENT DE PLAIES PROFONDES, PANSEMENTS A BASE DE GELS MOUSSANTS HYDROPHILES DE POLYURETHANE ET PROCEDE DE FABRICATION

(84) Benannte Vertragsstaaten:
**DE ES FR GB IT NL**

(30) Priorität: **02.10.1992 DE 4233289**
**17.03.1993 DE 4308347**
**17.03.1993 DE 4308445**

(43) Veröffentlichungstag der Anmeldung:
**09.08.1995 Patentblatt 1995/32**

(73) Patentinhaber: **Beiersdorf Aktiengesellschaft**
**20245 Hamburg (DE)**

(72) Erfinder:
• **KENNDOFF, Jochen**
**D-21149 Hamburg (DE)**
• **LENUCK, Vadim**
**D-22529 Hamburg (DE)**
• **SACHAU, Günther**
**D-25451 Quickborn (DE)**

(56) Entgegenhaltungen:
**EP-A- 0 057 839        EP-A- 0 147 588**
**EP-A- 0 163 150        EP-A- 0 190 814**
**WO-A-92/17518**

**Beschreibung**

Die vorliegende Erfindung betrifft Polyurethangelschäume, insbesondere solche für medizinische Anwendungen sowie Verfahren für ihre Herstellung.

Die vorliegende Erfindung betrifft weiterhin wässrige Flüssigkeiten absorbierende und bindende Polyurethangelschäume für medizinische Anwendungen sowie Verfahren zu ihrer Herstellung.

Die vorliegende Erfindung betrifft auch auf Trägermaterialien aufgebrachte, wässrige Flüssigkeiten absorbierende und bindende Polyurethangelschäume für medizinische Anwendungen, z.B. Wundverbände wie Wundpflaster und Heftpflaster sowie Verfahren für ihre Herstellung.

Die z.B. wässrige Flüssigkeiten absorbierenden und bindenden Polyurethangelschäume werden in der Folge auch als "Hydrogelschäume" oder "hydroaktive Gelschäume" bezeichnet. Statt "Polyurethangelschäume" können auch die Begriffe "Polyurethanschaumgele" oder "Gelschäume" und "Schaumgele" verwendet werden.

Hydrogele sind makromolekulare, natürliche oder synthetische Stoffe, die aufgrund eines hohen Gehaltes an hydrophilen Gruppen in der Lage sind, Wasser absorbtiv zu binden. Die Wasseraufnahmekapazität vieler Hydrogele beträgt das Mehrfache des Eigengewichtes der wasserfreien Substanz.

Hydrogele werden in vielfältiger Form in der Medizin eingesetzt. Besonders geeignet sind sie für die Wundversorgung. Sie können Wunden vor der Austrocknung schützen, Wundsekret aufsaugen, als Matrix für Wirkstoffe aller Art dienen und auch als Basis für die Besiedelung mit autologen oder heterologen Hautzellen dienen.

Hydrogele können unter anderem in Form von Sch'äumen verwendet werden. Schäume zur Versorgung von Hautwunden oder chirurgischen Wunden sind an sich bekannt. Hauptsächlich finden dabei Polyurethanschäume oder Kollagenschäume Verwendung.

Die Hydrogele des Standes der Technik haben jedoch verschiedene Nachteile:

Aufgrund ihrer Hydrophilie sind die meisten in Frage kommenden Stoffe wasserlöslich. Dies ist meist unerwünscht, weil derartige Produkte nicht formstabil sind. Außerdem lösen sich derartige Produkte in unerwünschter Weise am Einsatzort auf und stehen dann für den vorgesehenen Zweck nicht mehr zur Verfügung.

Andere Produkte zeichnen sich durch starke Polymervernetzung aus. Dadurch werden zwar manche der Nachteile der genannten Stoffklasse vermieden; die Quellbarkeit dieser Stoffe ist jedoch weitgehend eingeschränkt oder verloren.

Auch selbstklebende Gelschäume sind an sich bekannt. Diese lassen sich zwar im allgemeinen recht gut auf der Haut fixieren, haben aber meistens den Nachteil, daß ihre Wasseraufnahmekapazität stark eingeschränkt sind.

Weiterhin sind hydrophile Schäume aus Polyurethangelen bekannt. Die PCT-Anmeldung WO-88/01878 beschreibt selbstklebende Polyurethanschäume bzw. Polyurethanschaumgele, welche unter anderem einpolymerisierte Methacrylate enthalten können. Die Herstellung dieser Schaumgele erfolgt jedoch durch Zusatz von Wasser.

Polyurethangele auf der Basis einer Polyurethanmatrix und höhermolekularen Polyolen werden auch in EP-B-0 057 839 beschrieben. Selbsthaftende Flächergebilde aus Polyurethangelen sind aus EP-B-0 147 588 bekannt. Die in diesen beiden letztgenannten Schriften offenbarten Polyurethangele sind ungeschäumt. Die selbstklebenden Gele haben Isocyanatkennzahlen von 15 bis 70 (EP 147 588).

Hieraus geht hervor, daß die OH-Funktionalität stets im deutlichen Überschuß vorliegt.

In der EP-B-57839, die keine selbsthaftenden medizinischen Heftpflaster, sondern wirkstoffhaltige Gelmassen oder Abform- oder Eingußmassen zum Gegenstand hat, wurde das Schäumen solcher Massen mit Luft bereits erwähnt.

Die Patentanmeldung EP 0 453 286 beschreibt superabsorbierende Schaumkompositionen. Das Schäumen wird hier durch die Anwesenheit von Wasser, gegebenenfalls durch zusätzliche niedrig siedende organische Lösungsmittel erreicht.

EP 0 196 364 beschreibt hydrophile Polyurethanschäume, die mit wasserabsorbierenden Polymeren auf Basis eines Copolymers der Acrylsäure und Kaliumacetat gefüllt sein können und für medizinische Zwecke gedacht sind. Das Polyurethan wird auf Basis von MDI hergestellt. Der eingesetzte Polyether hat eine Mindestfunktionalität von 2 Hydroxylgruppen, bevorzugt jedoch 2 bis 3 Hydroxylgruppen. Das Verhältnis NCO/OH ist stöchiometrisch. Damit ist eine Variation der Eigenschaften nur sehr begrenzt möglich. Außerdem handelt es sich nicht um ein gelförmiges Polyurethan. Geschäumt kann mit Druckluft oder mit anderen, nicht mit dem Isocyanat reagierenden Gasen oder mit Hilfe von niedrig siedenden Lösungsmitteln werden. Die Mischung von Absorber mit Polyetherpolyol erfolgt etwa im Verhältnis 3 : 1. Der Schaum hat klebende Eigenschaften, die durch aluminisiertes Vlies gänzlich unterdrückt werden müssen, um zur Wundbehandlung eingesetzt werden zu können.

Die Patentanmeldung WO 92/17518 beschreibt hydrophile Schäume auf Polyurethangel-Basis, die durch einen Wasserzusatz geschäumt werden.

Es war nun Aufgabe der Erfindung, Hydrogelschäume zu entwickeln, die gegenüber dem Stand der Technik weniger Absorber enthalten, eine zusätzliche Entklebungsschicht grundsätzlich nicht benötigen sowie eine höhere Eigenschaftsflexibilität bezüglich des Kleb- und Saugverhaltens besitzen. Zudem soll auch das Streichen von extrem dünnen Schaumschichten möglich sein. Dabei sollen die Gelschäume einerseits auf der Haut haften, andererseits aber nicht mit der Wundoberfläche verkleben.

Diese Aufgabe wird gelöst durch selbstklebende, hydrophile Polyurethangelschäume, die erhältlich sind aus

1. einem Polyurethangel, welches

(A) 15-62 Gew.-%, vorzugsweise 20-57 Gew.-%, besonders bevorzugt 25-47 Gew.-%, bezogen auf die Summe aus (A) und (B), eines kovalent vernetzten Polyurethans als hochmolekulare Matrix und

(B) 85-38 Gew.-%, vorzugsweise 80-43 Gew.-%, besonders bevorzugt 75-53 Gew.-%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie gegebenenfalls

(C) 0-100 Gew.-%, bezogen auf die Summe aus (A) und (B), an Füll- und/oder Zusatzstoffen enthält, und welches erhältlich ist durch Umsetzung einer Mischung von

a) einem oder mehreren Polyisocyanaten,
b) einer oder mehreren Polyhydroxylverbindungen mit einem mittleren Molekulargewicht zwischen 1000 und

12000, und einer mittleren OH-Zahl zwischen 20 und 112,

c) gegebenenfalls Katalysatoren oder Beschleunigern für

die Reaktion zwischen Isocyanat- und Hydroxylgruppen sowie gegebenenfalls

d) aus der Polyurethanchemie an sich bekannten Füllund Zusatzstoffen,

wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität-der Polyhydroxylverbindung ($F_P$) zwischen 3 und 6 beträgt und die Isocyanatkennzahl (K) der Formel

$$K = \frac{300 \pm X}{(F_I \cdot F_P) - 1} + 7$$

gehorcht, in welcher $X \leq 120$, vorzugsweise $X \leq 100$, besonders bevorzugt $X \leq 90$ ist und die Kennzahl K bei Werten zwischen 15 und 70 liegt, wobei die angegebenen Mittelwerte von Molekulargewicht und OH-Zahl als Zahlenmittel zu verstehen sind,

2. einem Wasser absorbierendem Material und

3. einem nichtwäßrigem Schäumungsmittel.

Es war auch Aufgabe der Erfindung, Hydrogelschäume zu entwickeln, die gegenüber dem Stand der Technik weniger Absorber enthalten und eine zusätzliche Entklebungsschicht grundsätzlich nicht nötigen und zur Behandlung tiefer Wunden geeignet sind.

Das Schaumgel soll Selbstklebeeigenschaften haben, indem es gut auf sich selbst haften kann, so daß es der Wund- oder Körperhöhle gut angepaßt werden kann, andererseits aber nicht mit der Wundoberfläche verkleben. Zudem soll es sehr schnell große Mengen an Flüssigkeit binden können, welche sogar unter Druck nur zu einem geringen Teil wieder abgegeben werden können.

Diese Aufgabe wird gelöst durch hydrophile Polyurethangelschäume, die erhältlich sind aus

1. einem Polyurethangel, welches

(A) 35-62 Gew.-%, vorzugsweise 42-60 Gew.-%, besonders bevorzugt 49-57 Gew.-%, bezogen auf die Summe aus (A) und (B), eines kovalent vernetzten Polyurethans als hochmolekulare Matrix und

(B) 65-38 Gew.-%, vorzugsweise 58-40 Gew.-%, besonders bevorzugt 51-43 Gew.-%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie gegebenenfalls

(C) 0-100 Gew.-%, bezogen auf die Summe aus (A) und (B), an Füll- und/oder Zusatzstoffen enthält, und welches erhältlich ist durch Umsetzung einer Mischung von

a) einem oder mehreren Polyisocyanaten,
b) einer oder mehreren Polyhydroxylverbindungen mit einem mittleren Molekulargewicht zwischen 1000 und

12000, und einer mittleren OH-Zahl zwischen 20 und
112,

c) gegebenenfalls Katalysatoren oder Beschleunigern für

die Reaktion zwischen Isocyanat- und Hydroxylgruppen
sowie gegebenenfalls

d) aus der Polyurethanchemie an sich bekannten Füllund Zusatzstoffen,

wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxylverbindung ($F_P$) zwischen 3 und 6 beträgt und die Isocyanatkennzahl (K) der Formel

$$K = \frac{300 \pm X}{(F_I \cdot F_P) - 1} + 7$$

gehorcht, in welcher $X \leq 120$, vorzugsweise $X \leq 100$, besonders bevorzugt $X \leq 90$ ist und die Kennzahl K bei Werten zwischen 15 und 70 liegt, wobei die angegebenen Mittelwerte von Moiekulargewicht und OH-Zahl als Zahlenmittel zu verstehen sind,

2. einem Wasser absorbierendem Material und

3. einem nichtwäßrigem Schäumungsmittel.

Es war weiterhin Aufgabe der Erfindung, Wundverbände wie Wundpflaster und Heftpflaster mit Hydrogelschäumen für die Wundversorgung zu entwickeln, die gegenüber dem Stand der Technik weniger Absorber enthalten, eine zusätzliche Entklebungsschicht grundsätzlich nicht benötigen sowie eine höhere Eigenschaftsflexibilität bezüglich des Kleb- und Saugverhaltens aufweisen. Je nach Anwendungsgebiet sollte das geschäumte Material unterschiedliche Selbstklebeeigenschaften besitzen, z.B. um auf die Erfordernisse unterschiedlich empfindlicher Hauttypen besser eingehen zu können. Eine Haftung auf der feuchten Wundoberfläche soll nicht stattfinden. Ein weiteres Ziel ist, unterschiedliches Saugverhalten einstellen zu können, um damit auf spezielle Anforderungen spezifischer Wundtypen besser eingehen zu können. Die aufgesaugte Flüssigkeit soll auch unter leichtem Druck in der Wundauflage gehalten werden. Die Flüssigkeit soll zusätzlich möglichst senkrecht zur Wundfläche angesaugt und nicht in der Fläche verteilt werden, um z.B. Mazerationen an empfindlichen Wundrändern zu vermeiden. Weiterhin soll vor allem bei dünnen Schaumschichten die hohe Flexibilität der selbstklebenden Schäume genutzt werden, um auch den Sitz in Problemzonen zu gewährleisten.

Diese Aufgabe wird gelöst durch Wundverbände, die eine Polyurethanfolie als Träger und darauf aufgebrachte hydrophile Polyurethangelschäume umfassen, die erhältlich sind aus

1. einem Polyurethangel, welches

(A) 25-62 Gew.-%, vorzugsweise 30-60 Gew.-%, besonders bevorzugt 40-57 Gew.-%, bezogen auf die Summe aus (A) und (B), eines kovalent vernetzten Polyurethans als hochmolekulare Matrix und

(B) 75-38 Gew.-%, vorzugsweise 70-40 Gew.-%, besonders bevorzugt 60-43 Gew.-%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie gegebenenfalls

(C) 0-100 Gew.-%, bezogen auf die Summe aus (A) und (B), an Füll- und/oder Zusatzstoffen enthält, und welches erhältlich ist durch Umsetzung einer Mischung von

a) einem oder mehreren Polyisocyanaten,
b) einer oder mehreren Polyhydroxylverbindungen mit einem mittleren Molekulargewicht zwischen 1000 und 12000, und einer mittleren OH-Zahl zwischen 20 und 112,
c) gegebenenfalls Katalysatoren oder Beschleunigern für die Reaktion zwischen Isocyanat- und Hydroxylgruppen sowie gegebenenfalls
d) aus der Polyurethanchemie an sich bekannten Füllund Zusatzstoffen,

wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxylverbindung ($F_P$) zwischen 3 und 6 beträgt und die Isocyanatkennzahl (K) der Formel

$$K = \frac{300 \pm X}{(F_I \cdot F_P) - 1} + 7$$

gehorcht, in welcher $X \leq 120$, vorzugsweise $X \leq 100$, besonders bevorzugt $X \leq 90$ ist und die Kennzahl K bei Werten zwischen 15 und 70 liegt, wobei die angegebenen Mittelwerte von Molekulargewicht und OH-Zahl als Zahlenmittel zu verstehen sind,

2. einem Wasser absorbierenden Material und

3. einem nichtwäßrigen Schäumungsmittel.

Der Polyurethangelschaum dient als Wundauflage. Wundverbände gemäß der Erfindung sind auch Wundpflaster und Heftpflaster.

Die erfindungsgemäßen Polyurethangele können aus den an sich aus der Polyurethanchemie bekannten Ausgangsverbindungen nach an sich bekannten Verfahren hergestellt werden, wie sie z.B. in DE-OS 31 03 499, DE-OS 31 03 500 und EP 147 588 beschrieben werden. Wesentlich ist jedoch, daß bei der Auswahl der gelbildenden Komponenten die oben definierten Bedingungen eingehalten werden, da sonst anstelle von selbsthaftenden Gelen klebfreie, elastische Gele erhalten werden.

Erfindungsgemäß bevorzugte Polyhydroxylverbindungen sind Polyetherpolyole, wie sie in den oben genannten Deutschen Offenlegungsschriften ausführlich genannt sind.

Als Polyisocyanatkomponente sind sowohl (cyclo)aliphatische als auch aromatische Isocyanate geeignet. Bevorzugte (cyclo)aliphatische Polyisocyanate sind 1,6-Hexamethylen-diisocyanat sowie dessen Biurete und Trimerisate bzw. hydrierte Diphenylmethandiisocyanat ("MDI")-Typen. Bevorzugte aromatischen Polyisocyanate sind solche, die durch Destillation erhalten werden, wie MDI-Gemische aus 4,4'- und 2,4'-Isomeren oder 4,4'-MDI, sowie Toluylendiisocyanat ("TDI")-Typen. Die (cyclo)aliphatischen und die aromatischen Isocyanate können aufgrund von Modifizierungen, z.B. Biuretisierung, Trimerisierung oder Präpolymerisierung, auch höherfunktionelle Anteile beinhalten.

Die Diisocyanate können insbesondere z.B. aus der Gruppe der unmodifizierten aromatischen oder aliphatischen Diisocyanate oder aber aus durch Präpolymerisierung mit Aminen, Polyolen oder Polyetherpolyolen gebildeten modifizierten Produkten gewählt werden. Bevorzugt sind 4,4'-Diisocyanatodiphenylmethan, oder aber aus durch Präpolymerisierung mit Polyolen oder Polyetherpolyolen gebildete modifizierte Produkte. Als sehr günstig hat sich beispielsweise durch Präpolymerisierung mit Tripropylenglycol verflüssigtes 4,4'-Diisocyanatodiphenylmethan erwiesen. Bevorzugt werden ein oder mehrere Polyetherpolyole verwendet, die durch Anlagerung von Propylenoxid und gegebenenfalls Ethylenoxid an übliche Startermoleküle erhältlich sind und wie sie z.B. in EP 147 588 beschrieben sind.

Bevorzugte Polyether-Polyole sind in der nachfolgenden Tabelle zusammengestellt. Sie wurden durch Anlagerung von Propylenoxid und gegebenenfalls Ethylenoxid an die angegebenen Startermoleküle in an sich bekannter Weise hergestellt.

| Polyol Nr. | Propylenoxid % | Ethylenoxid % | Startermolekül | OH-Zahl | OH-Funktionalität |
|---|---|---|---|---|---|
| 1 | 80 | 20 | PET | 36 | 4 |
| 2 | 100 | - | Sorbit | 46 | 6 |
| 3 | 73 | 27 | Sorbit | 30 | 6 |
| 4 | 45 | 55 | TMP | 56 | 3 |
| 5 | 100 | - | PET | 72 | 4 |
| 6 | 100 | - | TMP | 56 | 3 |
| 7 | 90 | 10 | Sorbit | 83 | 6 |
| 8 | 100 | - | EDA | 61 | 4 |
| 9 | 83 | 17 | TMP | 35 | 3 |
| 10 | 100 | - | PET | 45 | 4 |

PET = Pentaerythrit

TMP = Trimethylolpropan

EDA = Ethylendiamin

Die Startermoleküle für die Polyetherpolyole sind z.B. Pentaerythrit, Sorbit, Trimethylolpropan oder Ethylendiamin. Besonders vorteilhaft werden die Katalysatoren oder Beschleuniger gewählt aus der Gruppe bestehend aus

- Organischen Säuren, insbesondere
  p-Toluolsulfonsäure,
  n-Butylphosphorsäure
- Organozinnverbindungen, einschließlich deren Salze organischer und anorganischer Säuren, insbesondere Zinnnaphthenat, Zinnbenzoat, Dibutylzinndioctoat, Dibutylzinndiacetat, Zinn-II-ethylhexoat und Dibutylzinndiacetat
- Eisensalzen höherer Fettsäuren, insbesondere Eisenstearat
- Aminen, zum Beispiel Isophorondiamin, Methylendianilin, Imidazole
- tertiären Aminen, insbesondere Trialkylamine, wobei die Alkylreste jeweils vorteilhaft 2 - 6 Kohlenstoffatome haben.

Erfindungsgemäß werden die Ausgangskomponenten so gewählt, daß im gelbildenden Reaktionsgemisch die mittlere NCO-Funktionalität zwischen 2 und 4, die mittlere Polyol-Funktionalität zwischen 3 und 6 und die Isocyanatkennzahl zwischen 15 und 70, vorzugsweise zwischen 18 und 55, besonders bevorzugt zwischen 20 und 45, liegen.

Die Polyurethan-Gele können gegebenenfalls aus der Polyurethan-Chemie an sich bekannte Zusatzstoffe enthalten, wie z.B. Füllstoffe und Kurzfasern auf anorganischer oder organischer Basis, Metallpigmente, oberflächenaktive Substanzen oder flüssige Streckmittel wie Substanzen mit einem Siedepunkt von über 150°C.

Als organische Füllstoffe seien beispielsweise Schwerspat, Kreide, Gips, Kieserit, Soda, Titandioxid, Ceroxid, Quarzsand, Kaolin, Ruß und Mikrohohlkugeln genannt.

An organischen Füllstoffen können z.B. Pulver auf Basis von Polystyrol, Polyvinylchlorid, Harnstoff-Formaldehyd und Polyhydrazodicarbonamid eingesetzt werden. Als Kurzfasern kommen z.B. Glasfasern von 0,1 - 1 mm Länge oder Fasern organischer Herkunft, wie z.B. Polyester- oder Polyamidfasern, in Frage. Metallpulver, wie z.B. Eisenoder Kupferpulver, können ebenfalls bei der Gelbildung mitverwendet werden. Um den erfindungsgemäßen Gelen die gewünschte Färbung zu verleihen, können die bei der Einfärbung von Polyurethanen an sich bekannten Farbstoffe oder Farbpigmente auf organischer oder anorganischer Basis verwendet werden, wie z.B. Eisenoxid- oder Chromoxidpigmente, Pigmente auf Phthalocyanin- oder Monoazo-Basis. Als oberflächenaktive Substanzen seien z.B. Cellulosepulver, Aktivkohle, und Kieselsäurepräparate genannt.

Als flüssige Streckmittel können beispielsweise Ethylstearat, Laurinsäurehexylester, Isopropoylmyristat, Isopropylpalmitat oder Dodecylsulfonsäureester verwendet werden.

Weiterhin können als flüssige Streckmittel auch höhermolekulare Polyole eingesetzt werden, deren Hydroxylgruppen verethert, verestert oder urethanisiert sind, sowie Paraffinöle und Silikonöle.

Der Gehalt an Füllstoffen und Streckmitteln in dem Gel kann vorzugsweise bis zu 50 Gew.-%, bezogen auf das Gesamtgewicht des Gels, betragen.

Zur Modifizierung der Hafteigenschaften der Gele können gegebenenfalls Zusätze von polymeren Vinylverbindungen, Polyacrylaten und sonstigen in der Klebstoff-Technik üblichen Copolymeren bzw. auch Klebemittel auf Naturstoffbasis bis zu einem Gehalt von 10 Gew.-%, bezogen auf das Gewicht der Gelmasse, zugegeben werden.

Die Füll- und Zusatzstoffe können auch aus den üblichen Substanzklassen gewählt werden. Insbesondere sind vorteilhaft Farbstoffe, Pigmente Lichtschutzmittel, Konservierungsmittel, Duftstoffe, antimikrobiell wirksame Substanzen, sonstige Wirkstoffe wie beispielsweise kühlend wirkende Substanzen (z.B. Menthol) oder solche, die die Durchblutung fördern oder ein Wärmegefühl erzeugen.

Bevorzugte Wasser absorbierende Materialien sind als Superabsorber bekannte Wasser absorbierende Salze von Polyacrylaten und deren Copolymeren, insbesondere die Natrium- oder Kaliumsalze. Sie können unvernetzt oder vernetzt sein und sind auch als Handelsprodukte erhältlich. Insbesondere sind solche Produkte geeignet, wie sie in der DE-A-37 13 601 offenbart werden und auch Superabsorber der neuen Generation mit nur noch geringen Anteilen an austrockenbarem Wasser und hohem Quellvermögen unter Druck.

Bevorzugte Produkte sind schwach vernetzte Polymerisate auf der Basis Acrylsäure/Natriumacrylat. Solche Natrium-polyacrylate sind als Favor 922-SK (Chemische Fabrik Stockhausen GmbH, Deutschland) erhältlich.

Weitere Absorber, z.B. Carboxymethylcellulose und Karaya sind ebenfalls geeignet.

Hydrophile Polyurethangelschäume

Für die hydrophilen Polyurethangelschäume werden die im folgenden angegebenen Mengen und Maßnahmen bevorzugt.

Die Gewichtsmenge des Wasser absorbierenden Materials kann bis zum 1,5-fachen der Gewichtsmenge der Polyhydroxyverbindung betragen. Bevorzugt beträgt die Gewichtsmenge des Wasser absorbierenden Materials 100 bis 2 Gew.-%, vorteilhaft auch 20 bis 70 Gew.-%, insbesondere 35 bis 60 Gew.-%, bezogen auf das Gewicht der Polyhydroxylverbindung.

Das Wasser absorbierende Material liegt vorzugsweise in fein gemahlener Form vor, insbesondere wenn dünne Schaumschichten gewünscht werden. Die Teilchengröße der Wasser absorbierenden Materialien, d.h. der Durchmesser des überwiegenden Anteils der Teilchen dieses Materials liegt vorzugsweise unter 300 µm, bevorzugt unter 200 µm, insbesondere aber unter 100µm. Bevorzugt beträgt die Teilchengröße 1 bis 100 µm, insbesondere 1 bis 70 µm.

Als nichtwäßriges Schäumungsmittel können wasserfreie oder weitgehend wasserfreie, vorzugsweise inerte Gase oder niedrig siedende Lösungsmittel verwendet werden, die sich dann in den Polyurethangelschäumen in fein verteilter Form befinden. Es können auch Gemische dieser Schäumungsmittel verwendet werden. Besonders bevorzugt werden Luft, Stickstoff oder Kohlendioxid oder Gemische dieser Gase, insbesondere aber Stickstoff. Eine zusätzliche Schäumung durch einen Wasserzusatz ist nicht erforderlich. Geeignete Lösungsmittel sind niedrigsiedende Lösungsmittel wie Ester, Ketone, Alkane und chlorierte Kohlenwasserstoffe, z.B. Alkylacetate wie Ethylacetat oder Trichlorfluormethan, Dichlordifluormethan, Methylenchlorid sowie die Treibgas-Ersatzstoffe.

Der Schäumungsgrad läßt sich durch die eingearbeiteten Mengen an Schäumungsmittel in weiten Grenzen variieren. So läßt sich die Dichte des Schaumes auf Werte von etwa 0,15 - 1,1 g/cm$^3$ einstellen. Dieser Dichtebereich ist für die Wundbehandlung geeignet.

Vorzugsweise sind alle verwendeten Ausgangsstoffe wasserfrei oder weitgehend wasserfrei oder als großtechnische Produkte wasserarm. Geeignet sind aber auch wasserhaltige Stoffe, in denen das Wasser so gebunden ist, daß es nicht reagiert.

Vorteilhaft sind die erfindungsgemäßen Schäume erhältlich aus

| 20 | 95 | Gew.-% Polyhydroxylverbindung |
|--------|----|-------------------------------|
| 1 | 60 | Gew.-% Polyisocyanat |
| 5 | 60 | Gew.-% Superabsorber |
| 0.0001 | 10 | Gew.-% Beschleuniger |

und dem nichtwäßrigen Schäumungsmittel in einer Menge, die Dichten von 0,15 bis 1,1 g/cm$^3$ ergibt.

Bevorzugt sind die erfindungsgemäßen Schäume erhältlich aus

| 50 | 70 | Gew.-% Polyhydroxylverbindung |
|-------|----|-------------------------------|
| 2 | 25 | Gew.-% Polyisocyanat |
| 5 | 40 | Gew.-% Superabsorber |
| 0.001 | 1 | Gew.-% Beschleuniger |

und dem nichtwäßrigen Schäumungsmittel in einer Menge, die Dichten von 0,3 bis 1,0 g/cm$^3$ ergibt.

Vorteilhaft ist es, Gewichts-Verhältnisse von Polyhydroxylverbindung : Polyisocyanat von (5 - 35) : 1 zu wählen, insbesondere etwa (10 - 25) : 1. Die Topfzeit als Maß für die Verarbeitbarkeit dieser Massen liegt zwischen 0.5 und 30 Minuten.

Die eingesetzten Polyetherpolyole können beispielsweise Handelsprodukte sein wie Levagel VP SN 100 und als Diisocyanate haben sich Handelsprodukte wie Desmodur PF, Desmodur N 100, IPDI und Desmodur W als geeignet erwiesen (Handelsprodukte der Bayer AG).

Für die Beschleuniger, so das Dibutylzinndilaurat, ist Desmorapid Z/SN, für Zinn-II-ethylhexoat Desmorapid SO ein vorteilhaftes Handelsprodukt (Bayer AG).

Gegenstand der Erfindung ist auch das Verfahren zur Herstellung der erfindungsgemäßen Polyurethanschäume, das dadurch gekennzeichnet ist, daß man

1. die vorstehend unter 1. genannten und definierten Komponenten (A), (B) und (C) des Polyurethangeles

2. ein Wasser absorbierendes Material und

3. ein nichtwäßriges Schäumungsmittel

vereinigt und miteinander mischt und schäumt, wobei Gase während des Vermischens eingebracht, insbesondere eingerührt oder eingeschlagen werden und eingebrachte Lösungsmittel durch Verdampfen in der Masse die Schäumung bewirken, wozu die Masse gegebenenfalls erwärmt wird.

Die Erwärmung erfolgt vorzugsweise in dem sich gegebenenfalls anschließenden Härtungsprozeß, insbesondere bei Temperaturen von 20° bis 140°C und Zeiten von 24 Stunden bis zu einigen Sekunden.

Die Herstellung der Gele kann insbesondere gemäß EP 147 588 auf verschiedene Weise erfolgen.

Man kann z.B. nach dem one-shot- oder dem Prepolymer-Verfahren arbeiten. Beim one-shot Verfahren werden alle Komponenten, d.h. Polyole, Di- und/oder Polyisocyanate, die Isocyanat-Polyadditionsreaktion beschleunigende Katalysatoren und gegebenenfalls Füll- und Zusatzstoffe und die übrigen Komponenten auf einmal zusammengegeben und intensiv miteinander vermischt.

Beim Prepolymer-Verfahren sind zwei Arbeitsweisen möglich. entweder stellt man zunächst ein Isocyanat-Prepolymer her, indem man einen entsprechenden Anteil der Polyolmenge mit der gesamten, für die Gelbilding vorgesehenen Isocyanatmenge umsetzt, und fügt dann dem erhaltenen Prepolymer die restliche Menge an Polyol sowie gegebenenfalls Füll -und Zusatzstoffe und die übrigen Komponenten zu und mischt intensiv. Oder man setzt die gesamte, für die Gelbildung vorgesehene Menge an Polyol mit einem Teil der Isocyanatmenge zu einem Hydroxyl-Prepolymer um und mischt anschließend die restliche Menge an Isocyanat zu.

Eine erfindungsgemäß besonders vorteilhafte Arbeitsweise ist eine Variante aus dem one-shot-Verfahren und dem Hydroxyl-Prepoylmer-Verfahren. Hierbei werden das Polyol bzw. Polyolgemisch, gegebenenfalls die Füll- und Zusatzstoffe und die übrigen Komponenten der Katalysator und zwei verschiedene Diisocyanate in einem Schuß zusammengegeben und intensiv vermischt, wobei ein Diisocyanat aliphatischer Natur ist. Man kann davon ausgehen, daß durch die stark unterschiedliche Reaktivität der beiden Diisocyanate zunächst ein Hydroxylprepolymer entsteht, das sodann innerhalb von Minuten mit dem anderen Diisocyanat unter Gelbildung reagiert.

Bei diesen Verfahrensweisen kann die Förderung, Dosierung und Mischung und Schäumung der Einzelkomponenten und der übrigen Komponenten oder Komponentengemische mit den für den Fachmann in der Polyurethan-Chemie an sich bekannten Vorrichtungen erfolgen.

Das Schäumen mit einem nichtwäßrigen Schäumungsmittel vorzugsweise durch Einrühren oder Einschlagen von Luft ist an sich bekannt, bietet aber hier im speziellen besondere Vorteile:

Die erfindungsgemäßen, vorteilhaften Polyurethangele können leicht zwischen gering und sehr stark klebend durch Variationen des Verhältnisses von NCO/OH gesteuert werden. Diese exakte Steuerung ist in einfacher Weise nur dann möglich, wenn im wesentlichen wasserfrei gearbeitet wird. Der Grund hierfür liegt in der extrem hohen Wasserabsorbtionsfähigkeit der Superabsorber, da zugesetztes Wasser, das für den Schäumungsprozeß zur Verfügung stehen muß, in einer unkontrollierten Weise dem Prozeß entzogen werden kann und wird, so daß es ungleich schwieriger ist, die Produkteigenschaften gezielt einzustellen.

Das erfindungsgemäße Schaummaterial erfüllt alle gestellten Anforderungen.

Überraschenderweise zeigen die erfindungsgemäßen Gelschäume die Eigenschaft, daß sie im feuchten Wundmilieu klebende Eigenschaften vollständig verlieren. Außerdem ist es leicht möglich, unterschiedliches Ansaugverhalten zu steuern. Auf der unverletzten Haut zeigen die erfindungsgemäßen Schäume dagegen die erwünschten selbstklebenden Eigenschaften.

Insbesondere ist es möglich, die Hafteigenschaften der erfindungsgemäßen Schäume durch das Verhältnis von Polyol zu Isocyanat zu steuern.

Insbesondere das Saugverhalten wird durch Schäumen und den Zusatz von wasserabsorbierenden Mitteln verbessert. Zusätzlich kann dadurch die Ansauggeschwindigkeit bei gleichbleibender Gesamtbindekapazität gesteuert werden. Beispielsweise erfolgt bei stärkerer Schäumung und/oder höherem Superabsorbergehalt eine schnellere Aufnahme von Wundsekreten.

Die Schäume können nach einer vorteilhaft etwa 0,5 - 30 Minuten messenden Topfzeit zu flächigen Gebilden ausgegossen oder ausgestrichen werden. Auf diese Weise sind Schaumdicken von 0,015 mm bis 15 cm problemlos erreichbar. Durch den Einsatz von fein gemahlenem Absorber sind beim Verstreichen dünne Masseaufträge bis zu 15 g / m² gut herstellbar. Es ist aber auch möglich und vorteilhaft, Gegenstände aus den erfindungsgemäßen Hydrogelschäumen anzufertigen, welche nicht flächig, sondern ausgeprägt raumfüllend sind, beispielsweise durch übliche Gußverfahren.

Die entstehenden Schäume sind offenporige Schäume. Es ist also nicht notwendig, sie durch Schnittverfahren in solche umzuwandeln.

Vorteilhaft können die erfindungsgemäßen Hydrogelschäume nach an sich bekannten Verfahren auch auf flächige Träger, z.B. Gewebe, Gewirke, Vliese oder Folien aufgetragen werden. Die erhaltenen Produkte sind ebenfalls Gegenstand der Erfindung.

Die in diesen erfindungsgemäßen selbsthaftenden Flächengebilden enthaltenen Trägermaterialien können unterschiedlichster Herkunft sein, d.h. Materialien auf Basis von natürlichen, halb- oder vollsynthetischen Rohstoffen sowie organischen oder anorganischen Urspungs sind verwendbar. Beispielsweise lassen sich Kunststoff- und Metallfolien, Matten, Vliese, Gewirke, Gestricke oder Gewebe aus organischem oder anorganischem Fasermaterial, Papier und Schaumstoff-Folien oder auch Kombinationen aus diesen Trägermaterialien einsetzen. Für die medizinische Anwendung bevorzugt sind luft- und feuchtigkeitsdurchlässige Flächengebilde, z.B. mikro- und makroporöse Kunststoffolien und Vliese, sowie elastische textile Trägermaterialien, insbesondere Stretchgewebe, und Mullbinden. Besonders bevorzugt werden Polyurethanfolien, insbesondere die nachstehend beschriebenen.

Gegenstand der Erfindung ist ferner ein Verfahren zur Herstellung selbsthaftender Flächengebilde auf Basis von mit Polyurethan-Gelschäumen beschichteten Trägermaterialien; das Verfahren ist dadurch gekennzeichnet, daß man die oben definierten Schäume oder zur Schaumgelbildung befähigten Reaktionsgemische auf die Oberfläche eines Trägermaterials aufbringt, z.B. nach Direktverfahren oder Umkehrverfahren durch Gießen oder Rakeln, wobei die Oberfläche mit dem gelbildenden Reaktionsgemisch gegebenenfalls nur teilweise bedeckt wird. Die Schichtdicke des Gelschaumes kann z.B. zwischen 0,015 mm und 150 mm, vorzugsweise zwischen 0,1 mm und 50 mm, besonders bevorzugt zwischen 0,1 mm und 6 mm, liegen.

Die Herstellung der erfindungsgemäßen Flächengebilde kann kontinuierlich oder diskontinuierlich erfolgen. Die Arbeitsweise hängt von den vorgegebenen, mit einer Haftschicht zu versehenden Flächengebilden ab. Wenn man bereits zugeschnittene Trägermaterialien vorliegen hat, ist oftmals eine diskontinuierliche Arbeitsweise vorteilhaft. Die kontinuierliche Arbeitsweise empfiehlt sich bei der Beschichtung von Trägermaterialien, die in endloser Form, z.B. als Rollenware, vorliegen. Der Auftrag der Schaum-Haftschicht auf das Trägermaterial kann dabei direkt oder nach dem Umkehrverfahren erfolgen. Das Reaktionsgemisch läßt sich bei den genannten Verfahren auch, bevor es durch die Reaktion erstarrt, rakeln.

Obwohl die Kohäsion der erfindungsgemäßen Schäume gut ist, kann eine Nachbehandlung der erfindungsgemäßen Schäume und Flächengebilde durch die Bestrahlung mit gamma-Strahlen, wodurch die Kohäsion in der Gelschicht verbessert wird, vorteilhaft sein.

Obwohl es, wie erwähnt, möglich ist, die Hafteigenschaften der erfindungsgemäßen Schäume zu steuern, so daß die erhaltenen Schäume oder die damit beschichteten Träger gut selbstklebend sein können, können sie vorteilhaft auf einer der beiden oder auch beiden Großflächen mit einer üblichen Selbstklebemasse beschichtet werden.

Gegenstand der Erfindung ist auch die Verwendung der erfindungsgemäßen Polyurethangelschäume oder der damit hergestellten selbsthaftenden Flächengebilde zur Herstellung von Mitteln für die medizinische Behandlung von Defektwunden bzw. zu deren Prophylaxe, als Heftpflaster oder Wundverband oder Wundpflaster, Bandage oder Binde sowie als Schutz und Polstermaterial, insbesondere Mitteln zur Prophylaxe.

Der fertige Schaum, vorzugsweise die vorabbeschriebenen flächigen Gebilde, vorteilhaft aber auch die mit Hydrogelschaum beschichteten Träger, können beispielsweise dazu dienen, Oberflächenwunden oder chirurgische Wunden zu versorgen.

Ein besonders vorteilhaftes Anwendungsgebiet ist die Versorgung tiefer Wunden. Insbesondere deshalb, weil die erfindungsgemäßen Schäume aufeinander haften können. Eine oder mehrere dünne Schaumschichten, die einzeln sehr bequem in geeignete Formen zu schneiden sind, können z.B. modelliert und der Wunde angepaßt werden, ohne daß es weiterer Fixierungshilfsmittel bedürfte. Dadurch wird die gleiche Wirkung erzielt wie mit dicken Schaumschichten.

Die gute kohäsive Eigenschaft der erfindungsgemäßen Schäume läßt sie auch gut zur Verwendung auf kohäsiven Binden geeignet sein. Dazu werden sie z.B. auf elastische oder starre Träger-Materialien aufgetragen.

Im Rahmen der vorliegenden Anmeldung sind, soweit nicht anders angegeben, alle Mengen und Prozentangaben auf das Gewicht und die Gesamtzusammensetzung der Zubereitung bezogen.

Behandlung tiefer Wunden

Für die Polyurethangelschäume, die insbesondere zur Behandlung tiefer Wunden geeignet sind, werden die im folgenden angegebenen Mengen und Maßnahmen bevorzugt.

Die Gewichtsmenge des Wasser absorbierenden Materials kann bis zum 1,5-fachen der Gewichtsmenge der Polyhydroxyverbindung betragen. Bevorzugt beträgt die Gewichtsmenge des Wasser absorbierenden Materials 5 bis 67 Gew.-%, vorteilhaft auch 10 bis 52 Gew.-X, insbesondere 15 bis 40 Gew.-%, bezogen auf das Gewicht der Polyhydroxylverbindung. Besonders bevorzugt beträgt diese Gewichtsmenge auch 24 bis 67 Gew.-%.

Das Wasser absorbierende Material liegt vorzugsweise in fein gemahlener Form vor. Die Teilchengröße der Wasser absorbierenden Materialien, d.h. der Durchmesser des überwiegenden Anteils der Teilchen dieses Materials liegt vorzugsweise unter 300 µm, bevorzugt unter 200 µm, insbesondere aber unter 100µm. Bevorzugt beträgt die Teilchengröße 10 bis 70 µm.

Als nichtwäßriges Schäumungsmittel können wasserfreie, vorzugsweise inerte Gase oder niedrig siedende Lösungsmittel verwendet werden, die sich dann in den Polyurethangelschäumen in fein verteilter Form befinden. Es können auch Gemische dieser Schäumungsmittel verwendet werden. Besonders bevorzugt werden Luft, Stickstoff oder Kohlendioxid oder Gemische dieser Gase, insbesondere aber Stickstoff. Eine zusätzliche Schäumung durch einen Wasserzusatz ist nicht erforderlich. Geeignete Lösungsmittel sind niedrigsiedende Lösungsmittel wie Ester, Ketone, Alkane und chlorierte Kohlenwasserstoffe, z.B. Alkylacetate wie Ethylacetat oder Trichlorfluormethan, Dichlordifluormethan, Methylenchlorid sowie die Treibgas-Ersatzstoffe.

Der Schäumungsgrad läßt sich durch die eingearbeiteten Mengen an Schäumungsmittel in weiten Grenzen variieren. So läßt sich die Dichte des Schaumes auf Werte von etwa 0,15 - 1,1 $g/cm^3$ einstellen. Dieser Dichtebereich ist für die Wundbehandlung geeignet. Bevorzugt sind Dichten zwischen 0,30 und 0,65 $g/cm^3$, insbesondere Dichten zwischen 0,45 und 0,6 $g/cm^3$.

Zahlenwerte für die Schäumungsmittelmengen sind selbst in weiten Grenzen nicht sinnvoll anzugeben, da diese stets in direkter Abhängigkeit von der beispielsweise pro Minute ausgestoßenen Menge an zu schäumendem Material stehen, außerdem Dichteschwankungen des zu schäumenden Materials eingehen, sowie Löslichkeits- bzw. Ausgasungsphänomene in Abhängigkeit von den Rohstoffqualitäten produktionsbedingt möglich sind.

Daher ist grundsätzlich durch Ausprobieren die Dichte einzustellen, was bei geeigneter technischer Ausrüstung innerhalb von Minuten erfolgen kann und dem Fachmann keine Schwierigkeiten bereitet.

Je nach Qualität der eingesetzten Rohstoffe werden, bezogen auf die eingesetzte Gewichtsmenge an Polyhydroxylverbindungen vorzugsweise 4,7 - 6,9 Gew.-%, bevorzugt 5,0 - 6,4 Gew.-%, insbesondere aber 5,4 - 5,9 Gew.-% an Isocyanat als Vernetzer zugesetzt.

Der Gewichtsanteil an Polyhydroxylverbindung bezogen auf das Gesamtgewicht der eingesetzten Rohstoffe kann zwischen 60 und 80 Gew.-%, bevorzugt zwischen 63 und 77 Gew.-%, nsbesondere aber zwischen 65 und 75 Gew.-% liegen.

Der Gewichtsanteil an Superabsorber bezogen auf das Gesamtgewicht der eingesetzten Rohstoffe kann zwischen 20 und 40, bevorzugt zwischen 23 und 37 Gew.-%, insbesondere aber zwischen 25 und 35 Gew.-% betragen.

Vorzugsweise sind alle verwendeten Ausgangsstoffe wasserfrei oder weitgehend wasserfrei oder als großtechnische Produkte wasserarm. Geeignet sind aber auch wasserhaltige Stoffe, in denen das Wasser so gebunden ist, daß es nicht reagiert.

Vorteilhaft sind die erfindungsgemäßen Schäume erhältlich aus

| 40 | 95 | Gew.-% Polyhydroxylverbindung |
|--------|-----|-------------------------------|
| 1 | 20 | Gew.-% Polyisocyanat |
| 1 | 60 | Gew.-% Superabsorber |
| 0.0001 | 10 | Gew.-% Beschleuniger |

und dem nichtwäßrigen Schäumungsmittel in einer Menge, die Dichten von 0,15 bis 1,1 $g/cm^3$ ergibt, bevorzugt zwischen 0,3 bis 0,65 $g/cm^3$, besonders bevorzugt zwischen 0,45 und 0,60 $g/cm^3$.

Bevorzugt sind die erfindungsgemäßen Schäume erhältlich aus

| 55 | 85 | Gew.-% Polyhydroxylverbindung |
|----|----|-------------------------------|
| 2 | 10 | Gew.-% Polyisocyanat |
| 20 | 40 | Gew.-% Superabsorber |

(fortgesetzt)

| 0.001 | 1 | Gew.-% Beschleuniger |
|---|---|---|

und dem nichtwäßrigen Schäumungsmittel in einer Menge, die Dichten von 0,45 bis 0,6 g/cm$^3$ ergibt.

Vorteilhaft ist es, Gewichts-Verhältnisse von Polyhydroxylverbindung : Polyisocyanat von (10 - 35) : 1 zu wählen, insbesondere etwa (15 - 25) : 1. Die Topfzeit als Maß für die Verarbeitbarkeit dieser Massen liegt zwischen 0,5 und 30 Minuten.

Die eingesetzten Polyetherpolyole können beispielsweise Handelsprodukte sein wie Levagel VP SN 100 und als Diisocyanate haben sich Handelsprodukte wie Desmodur PF, Desmodur N 100, IPDI und Desmodur W als geeignet erwiesen (Handelsprodukte der Bayer AG).

Für die Beschleuniger, so das Dibutylzinndilaurat, ist Desmorapid Z/SN, für Zinn-II-ethylhexoat Desmorapid SO ein vorteilhaftes Handelsprodukt (Bayer AG).

Gegenstand der Erfindung ist auch das Verfahren zur Herstellung der erfindungsgemäßen Polyurethanschäume, das dadurch gekennzeichnet ist, daß man

1. die vorstehend unter 1. genannten und definierten Komponenten (A), (B) und (C) des Polyurethangeles,

2. ein Wasser absorbierendes Material und

3. ein nichtwäßriges Schäumungsmittel

vereinigt und miteinander mischt und schäumt, wobei Gäse während des Vermischens eingebracht, insbesondere eingerührt oder eingeschlagen werden und eingebrachte Lösungsmittel durch Verdampfen in der Masse die Schäumung bewirken, wozu die Masse gegebenenfalls erwärmt wird.

Die Erwärmung erfolgt vorzugsweise in dem sich gegebenenfalls anschließenden Härtungsprozeß, insbesondere bei Temperaturen von 20° bis 140°C und Zeiten von 24 Stunden bis zu einigen Sekunden.

Die Herstellung der Gele kann insbesondere gemäß EP 147 588 auf verschiedene Weise erfolgen.

Man kann z.B. nach dem one-shot- oder dem Prepolymer-Verfahren arbeiten. Beim one-shot Verfahren werden alle Komponenten, d.h. polyole, Di- und/oder Polyisocyanate, die Isocyanat-Polyadditionsreaktion beschleunigende Katalysatoren und gegebenenfalls Füll- und Zusatzstoffe und die übrigen Komponenten auf einmal zusammengegeben und intensiv miteinander vermischt.

Beim Prepolymer-Verfahren sind zwei Arbeitsweisen möglich. Entweder stellt man zunächst ein Isocyanat-Prepolymer her, indem man einen entsprechenden Anteil der Polyolmenge mit der gesamten, für die Gelbildung vorgesehenen Isocyanatmenge umsetzt, und fügt dann dem erhaltenen Prepolymer die restliche Menge an Polyol sowie gegebenenfalls Füll -und Zusatzstoffe und die übrigen Komponenten zu und mischt intensiv. Oder man setzt die gesamte, für die Gelbildung vorgesehene Menge an Polyol mit einem Teil der Isocyanatmenge zu einem Hydroxyl-Prepolymer um und mischt anschließend die restliche Menge an Isocyanat zu.

Eine erfindungsgemäß besonders vorteilhafte Arbeitsweise ist eine Variante aus dem one-shot-Verfahren und dem Hydroxyl-Prepoylmer-Verfahren. Hierbei werden das Polyol bzw. Polyolgemisch, gegebenenfalls die Füll- und Zusatzstoffe und die übrigen Komponenten der Katalysator und zwei verschiedene Diisocyanate in einem Schuß zusammengegeben und intensiv vermischt, wobei ein Diisocyanat aliphatischer Natur ist. Man kann davon ausgehen, daß durch die stark unterschiedliche Reaktivität der beiden Diisocyanate zunächst ein Hydroxylprepolymer entsteht, das sodann innerhalb von Minuten mit dem anderen Diisocyanat unter Gelbildung reagiert.

Bei diesen Verfahrensweisen kann die Förderung, Dosierung und Mischung und Schäumung der Einzelkomponenten und der übrigen Komponenten oder Komponentengemische mit den für den Fachmann in der Polyurethan-Chemie an sich bekannten Vorrichtungen erfolgen.

Das Schäumen mit einem nichtwäßrigen Schäumungsmittel vorzugsweise durch Einrühren oder Einschlagen von Luft oder Stickstoff ist an sich bekannt, bietet aber hier im speziellen besondere Vorteile:

Die erfindungsgemäßen, vorteilhaften Polyurethangelschäume können so leicht schwach auf Haut klebend und gut auf sich selbst haftend durch Variationen des Verhältnisses von Polyhydroxylverbindung zu Isocyanat eingestellt werden. Durch Erniedrigung der zugegebenen Isocyanatmenge, bezogen auf die Polyhydroxylverbindung, erhöht sich die Klebkraft und durch Erhöhung der zugegebenen Isocyanatmenge verringert sich die Klebkraft. Diese exakte Steuerung ist in einfacher Weise nur dann möglich, wenn im wesentlichen wasserfrei gearbeitet wird. Der Grund hierfür liegt in der extrem hohen Wasserabsorptionsfähigkeit der Superabsorber, da zugesetztes Wasser, das für den Schäumungsprozeß zur Verfügung stehen muß, in einer unkontrollierten Weise dem Prozeß entzogen werden kann und wird, so daß es ungleich schwieriger ist, die Produkteigenschaften gezielt einzustellen.

Das erfindungsgemäße Schaummaterial erfüllt alle gestellten Anforderungen, und saugt Wundflüssigkeit schnell und sicher auf.

Überraschenderweise haben die erfindungsgemäßen Gelschäume die Eigenschaft, daß sie im feuchten Wundmilieu klebende Eigenschaften gegenüber der Wundfläche vollständig verlieren. Außerdem zeigen sie ein schnelles Ansaugen bei hoher Aufnahmekapazität. Der Schaum ist dabei schwach klebend, haftet aber gut auf sich selbst, so daß der Schaum der Wunde oder der Körperöffnung entsprechend modelliert werden kann. Dabei kann die Modellierung reversibel sein und mehrfach umgestaltet werden, so daß das Material einer tiefen Wunde, Körperhöhle oder Körperöffnung ohne Probleme gut angepaßt werden kann. Dabei können auch mehrere Teile gestapelt, überlappend oder in anderer Form zusammengebracht werden und einer Wunde oder Öffnung gleich welcher Art, die größer ist als das einzelne Schaumprodukt, angepaßt werden. Sie lassen sich in einem Stück aus der Wundhöhle entfernen, da sie während der Anwendung miteinander verbunden bleiben.

Die Schäume können nach einer vorteilhaft etwa 0,5 - 30 Minuten messenden Topfzeit zu flächigen Gebilden ausgegossen oder ausgestrichen werden. Auf diese Weise sind Schaumdicken von 0,015 mm bis 15 cm problemlos erreichbar. Als vorteilhaft haben sich Schaumdicken von 2 bis 6 mm erwiesen. Es ist aber auch möglich und vorteilhaft, Gegenstände aus den erfindungsgemäßen Hydrogelschäumen anzufertigen, welche nicht flächig, sondern ausgeprägt raumfüllend sind, beispielsweise durch übliche Gußverfahren.

Die entstehenden Schäume sind offenporige Schäume. Es ist also nicht notwendig, sie durch Schnittverfahren in solche umzuwandeln.

Obwohl die Kohäsion der erfindungsgemäßen Schäume gut ist, kann eine Nachbehandlung der erfindungsgemäßen Schäume und Flächengebilde durch die Bestrahlung mit gamma-Strahlen, wodurch die Kohäsion in der Gelschicht verbessert werden kann, vorteilhaft sein. Zudem besteht die Möglichkeit, durch eingeschäumte, vollständig bedeckte flächige Gebilde wie z.B. Vliese, Gewebe und Gewirke in elastischer oder nicht elastischer Form, die gute Stabilität der Schäume weiter zu erhöhen.

Gegenstand der Erfindung ist auch die Verwendung der erfindungsgemäßen Polyurethangelschäume oder der damit hergestellten schwach klebenden und gut auf sich selbst haftenden Flächengebilde zur Herstellung von Mitteln für die medizinische Behandlung von tiefen Wunden, Defektwunden und Wundhöhlen. Auch ist ein Einsatz im zahnmedizinischen Bereich möglich, z.B. zum Binden von Speichel oder zum Trocknen oder Trockenhalten von Zahnhöhlen.

Besonders günstig ist die Versorgung tiefer Wunden deshalb, weil die erfindungsgemäßen Schäume aufeinander haften können. Eine oder mehrere dünne Schaumschichten, die einzeln sehr bequem in geeignete Formen zu schneiden sind, können z.B. modelliert und der Wunde angepaßt werden, ohne daß es weiterer Fixierungshilfsmittel bedürfte. Dadurch wird die gleiche Wirkung erzielt wie mit dicken Schaumschichten.

Im Rahmen der vorliegenden Anmeldung sind, soweit nicht anders angegeben, alle Mengen und Prozentangaben auf das Gewicht und die Gesamtzusammensetzung der Zubereitung bezogen.

Wundverbände

Für die Polyurethangelschäume, die insbesondere für Wundverbände geeignet sind, werden die im folgenden angegebenen Mengen und Maßnahmen bevorzugt.

Die Gewichtsmenge des Wasser absorbierenden Materials kann z.B. bis zum 1,5-fachen der Gewichtsmenge der Polyhydroxyverbindung betragen. Bevorzugt beträgt die Gewichtsmenge des Wasser absorbierenden Materials 2 bis 50 Gew.-%, vorteilhaft auch 5 bis 40 Gew.-%, insbesondere 10 bis 35 Gew.-%, bezogen auf das Gewicht der Polyhydroxylverbindung.

Das Wasser absorbierende Material liegt vorzugsweise in fein gemahlener Form vor, insbesondere wenn dünne Schaumschichten gewünscht werden. Die Teilchengröße der Wasser absorbierenden Materialien, d.h. der Durchmesser des überwiegenden Anteils der Teilchen dieses Materials liegt vorzugsweise unter 300 um, bevorzugt unter 200 um, besonders bevorzugt aber unter 100 μm, insbesondere unter 70 um. Bevorzugt beträgt die Teilchengröße 1 bis 70 um.

Als nichtwäßriges Schäumungsmittel können wasserfreie oder weitgehend wasserfreie, vorzugsweise inerte Gase oder niedrig siedende Lösungsmittel verwendet werden, die sich dann in den Polyurethangelschäumen in fein verteilter Form befinden. Es können auch Gemische dieser Schäumungsmittel verwendet werden. Besonders bevorzugt werden Luft, Stickstoff oder Kohlendioxid oder Gemische dieser Gase, insbesondere aber Stickstoff. Eine zusätzliche Schäumung durch einen Wasserzusatz ist nicht erforderlich. Geeignete Lösungsmittel sind niedrigsiedende Lösungsmittel wie Ester, Ketone, Alkane und chlorierte Kohlenwasserstoffe, z.B. Alkylacetate wie Ethylacetat oder Trichlorfluormethan, Dichlordifluormethan, Methylenchlorid sowie die Treibgas-Ersatzstoffe.

Der Schäumungsgrad läßt sich durch die eingearbeiteten Mengen an Schäumungsmittel variieren. So läßt sich die Dichte des Schaumes auf Werte von etwa 0,15 - 1,1 g/cm$^3$ einstellen. Dieser Dichtebereich ist für die Wundbehandlung geeignet. Je nach Bedarf sind unterschiedliche Dichten einstellbar. So kann beispielsweise ein Schaum hergestellt werden, der sehr schnell wässrige Flüssigkeiten aufsaugt. Hierfür besonders geeignet sind Schaumdichten von 0,30 bis 0,65 g/cm$^3$, insbesondere aber von 0,45 bis 0,6 g/cm$^3$ (a).

Für ein Schaumprodukt mit hoher Absorptionskapazität aber nur mäßig schnellem Ansaugverhalten sind Schaumdichten von 0,6 bis 0,9 g/cm$^3$, insbesondere aber von 0,65 bis 0,8 g/cm$^3$ vorteilhaft (b).

Für Produkte mit langsamen Ansaugverhalten sind bei hoher Kapazität Dichten von 0,8 bis 1,0 g/cm$^3$, insbesondere von 0,85 bis 0,95 g/cm$^3$ gut geeignet (c).

Vorzugsweise sind alle verwendeten Ausgangsstoffe wasserfrei oder weitgehend wasserfrei oder als großtechnische Produkte wasserarm. Geeignet sind aber auch wasserhaltige Stoffe, in denen das Wasser so gebunden ist, daß es nicht reagiert.

Vorteilhaft sind die erfindungsgemäßen Schäume erhältlich aus

| 20 | 95 | Gew.-% Polyhydroxylverbindung |
|---|---|---|
| 1 | 30 | Gew.-% Polyisocyanat |
| 1 | 60 | Gew.-% Superabsorber |
| 0.0001 | 10 | Gew.-% Beschleuniger |

und dem nichtwäßrigen Schäumungsmittel in einer Menge, die Dichten von 0,15 bis 1,1 g/cm$^3$ ergibt, insbesondere den vorstehend angegebenen Dichten, je nachdem, welches Saugverhalten erwünscht ist.

Bevorzugt sind daher die erfindungsgemäßen Schäume erhältlich aus

| 55 | 85 | Gew.-% Polyhydroxylverbindung |
|---|---|---|
| 2 | 15 | Gew.-% Polyisocyanat |
| 5 | 40 | Gew.-% Superabsorber |
| 0.001 | 1 | Gew.-% Beschleuniger |

und dem nichtwäßrigen Schäumungsmittel in einer solchen Menge, daß

(a) Dichten von 0,30 bis 0,65 g/cm$^3$, insbesondere aber 0,45 bis 0,6 g/cm$^3$
oder
(b) Dichten von 0,6 bis 0,9 g/cm$^3$, insbesondere aber 0,65 bis 0,8 g/cm$^3$
oder
(c) Dichten von 0,8 bis 1,0 g/cm$^3$, insbesondere aber 0,85 bis 0,95 g/cm$^3$ erhalten werden.

Diesen Dichtebereichen entsprechen die vorstehend unter (a) - (c) angegebenen Eigenschaften.

Zahlenwerte für die Schäumgsmittelmengen sind selbst in weiten Grenzen nicht sinnvoll anzuzeigen, da diese stets in direkter Abhängigkeit von der beispielweise pro Minute ausgestoßenen Menge an zu schäumendem Material stehen, außerdem Dichteschwankungen des zu schäumenden Materials eingehen, sowie Löslichkeits- bzw. Ausgasungsphänomene in Abhängigkeit von den Rohstoffqualitäten produktionsbedingt möglich sind.

Daher ist grundsätzlich durch Ausprobieren die Dichte einzustellen, was bei geeigneter technischer Ausrüstung innerhalb von Minuten erfolgen kann und dem Fachmann keine Schwierigkeiten bereitet.

Vorteilhaft ist es, Gewichts-Verhältnisse von Polyhydroxylverbindung : Polyisocyanat von (5 - 35) : 1 zu wählen, insbesondere etwa (10 - 25) : 1. Die Topfzeit als Maß für die Verarbeitbarkeit dieser Massen liegt zwischen 0,5 und 30 Minuten.

Die eingesetzten Polyetherpolycle können beispielsweise Handelsprodukte sein wie Levagel VP SN 100 und als Diisocyanate haben sich Handelsprodukte wie Desmodur PF, Desmodur N 100, IPDI und Desmodur W als geeignet erwiesen (Handelsprodukte der Bayer AG).

Für die Beschleuniger, so das Dibutylzinndilaurat, ist Desmorapid Z/SN, für Zinn-II-ethylhexoat Desmorapid SO ein vorteilhaftes Handelsprodukt (Bayer AG).

Gegenstand der Erfindung ist auch das Verfahren zur Herstellung der erfindungsgemäßen Polyurethanschäume, das dadurch gekennzeichnet ist, daß man

1. die vorstehend unter 1. genannten und definierten Komponenten (A), (B) und (C) des Polyurethangeles,
2. ein Wasser absorbierendes Material und
3. ein nichtwäßriges Schäumungsmittel

vereinigt und miteinander mischt und schäumt, wobei Gase während des Vermischens eingebracht, insbesondere eingerührt oder eingeschlagen werden und eingebrachte

Lösungsmittel durch Verdampfen in der Masse die Schäumung bewirken, wozu die Masse gegebenenfalls erwärmt wird.

Die Erwärmung erfolgt vorzugsweise in dem sich gegebenenfalls anschließenden Härtungsprozeß, insbesondere bei Temperaturen von 20° bis 140°C und Zeiten von 24 Stunden bis zu einigen Sekunden.

Die Herstellung der Gele kann insbesondere gemäß EP 147 588 auf verschiedene Weise erfolgen.

Man kann z.B. nach dem one-shot- oder dem Prepolymer-Verfahren arbeiten. Beim one-shot Verfahren werden alle Komponenten, d.h. Polyole, Di- und/oder Polyisocyanate, die Isocyanat-Polyadditionsreaktion beschleunigende Katalysatoren und gegebenenfalls Füll- und Zusatzstoffe und die übrigen Komponenten auf einmal zusammengegeben und intensiv miteinander vermischt.

Beim Prepolymer-Verfahren sind zwei Arbeitsweisen möglich. entweder stellt man zunächst ein Isocyanat-Prepolymer her, indem man einen entsprechenden Anteil der Polyolmenge mit der gesamten, für die Gelbildung vorgesehenen Isocyanatmenge umsetzt, und fügt dann dem erhaltenen Prepolymer die restliche Menge an Polyol sowie gegebenenfalls Füll- und Zusatzstoffe und die übrigen Komponenten zu und mischt intensiv. Oder man setzt die gesamte, für die Gelbildung vorgesehene Menge an Polyol mit einem Teil der Isocyanatmenge zu einem Hydroxyl-Prepolymer um und mischt anschließend die restliche Menge an Isocyanat zu.

Eine erfindungsgemäß besonders vorteilhafte Arbeitsweise ist eine Variante aus dem one-shot-Verfahren und dem Hydroxyl-Prepoylmer-Verfahren. Hierbei werden das Polyol bzw. Polyolgemisch, gegebenenfalls die Füll- und Zusatzstoffe und die übrigen Komponenten der Katalysator und zwei verschiedene Diisocyanate in einem Schuß zusammengegeben und intensiv vermischt, wobei ein Diisocyanat aliphatischer Natur ist. Man kann davon ausgehen, daß durch die stark unterschiedliche Reaktivität der beiden Diisocyanate zunächst ein Hydroxylprepolymer entsteht, das sodann innerhalb von Minuten mit dem anderen Diisocyanat unter Gelbildung reagiert.

Bei diesen Verfahrensweisen kann die Förderung, Dosierung und Mischung und Schäumung der Einzelkomponenten und der übrigen Komponenten oder Komponentengemische mit den für den Fachmann in der Polyurethan-Chemie an sich bekannten Vorrichtungen erfolgen.

Das Schäumen mit einem nichtwäßrigen Schäumungsmittel vorzugsweise durch Einrühren oder Einschlagen von Luft ist an sich bekannt, bietet aber hier im speziellen besondere Vorteile:

Die erfindungsgemäßen, vorteilhaften Polyurethangele können so leicht schwach klebend und gut auf sich selbst haftend bis hin zu sehr gut klebend durch Variationen des Verhältnisses von Polyhydroxylverbindung zur Isocyanat gesteuert werden. Durch Erniedrigung der zugegebenen Isocyanatmenge, bezogen auf die Polyhydroxylverbindung erhöht sich die Klebkraft und durch Erhöhung der zugegebenen Isocyanatmenge verringert sich die Klebkraft. Diese exakte Steuerung ist in einfacher Weise nur dann möglich, wenn im wesentlichen wasserfrei gearbeitet wird. Der Grund hierfür liegt in der extrem hohen Wasserabsorbtionsfähigkeit der Superabsorber, da zugesetztes Wasser, das für den Schäumungsprozeß zur Verfügung stehen muß, in einer unkontrollierten Weise dem Prozeß entzogen werden kann und wird, so daß es ungleich schwieriger ist, die Produkteigenschaften gezielt einzustellen.

Die erfindungsgemäßen Wundverbände mit dem erfindungsgemäßen Schaummaterial erfüllen alle gestellten Anforderungen.

Überraschenderweise zeigen die erfindungsgemäßen Gelschäume die Eigenschaft, daß sie im feuchten Wundmilieu klebende Eigenschaften vollständig verlieren. Außerdem ist es leicht möglich, ein schnelles, mittleres oder langsames Ansaugen bei hoher Aufnahmekapazität zu erhalten. Der Schaum ist dabei schwach oder stark selbstklebend, so daß er gut unterschiedlichen Hauttypen angepaßt werden kann. Sehr wichtig ist, daß das aufgesaugte Material praktisch nicht in der Fläche der Wundauflage verteilt wird.

Die Schäume können nach einer vorteilhaft etwa 0,5 - 30 Minuten messenden Topfzeit zu flächigen Gebilden ausgegossen oder ausgestrichen werden. Auf diese Weise sind Schaumdicken von 0,015 mm bis 15 cm problemlos erreichbar. Die Stärke des Matrials ist wiederum abhängig vom Einsatzzweck: soll pro Flächeneinheit viel Flüssigkeit aufgenommen werden, so wird ein entsprechend dickes Schaummaterial gestrichen. Ist nur ein geringfügiges Flüssigkeitsvolumen pro Flächeneinheit zu versorgen sind auch sehr dünne Schichten ausreichend. Der Freiheitsgrad "Dicke" ist erforderlich, da die aufgenommene Flüssigkeit im wesentlichen senkrecht zur Wundoberfläche aufgesaugt wird und nicht in der Fläche verteilt wird. Durch den Einsatz von fein gemahlenem Absorber sind beim Verstreichen dünne Masseaufträge bis zu 10 g / m$^2$ gut herstellbar. Es ist aber auch möglich und vorteilhaft, Gegenstände aus den erfindungsgemäßen Hydrogelschäumen anzufertigen, welche nicht flächig, sondern ausgeprägt raumfüllend sind, beispielsweise durch übliche Gußverfahren.

Die entstehenden Schäume sind offenporige Schäume. Es ist also nicht notwendig, sie durch Schnittverfahren in solche umzuwandeln.

Vorteilhaft können die erfindungsgemäßen Hydrogelschäume nach an sich bekannten Verfahren auf den flächigen Träger aufgetragen werden. Vorzugsweise wird eine Seite des Trägers mit einer erfindungsgemäßen Polyurethangelschaumschicht versehen.

Erfindungsgemäß lassen sich als Träger Polyurethanfolien einsetzen, da hierbei die hohe Flexibilität der Polyurethanhydrogelschäume durch das flexible Trägermaterial nicht behindert wird und somit auch im Gelenkbereich gut haftet. Solche Polyurethanfolien sind bekannt und im Handel erhältlich.

Sehr vorteilhaft kommen durch die offenporige Schaumstruktur auch Wasserdampfdurchlässigkeiten und Luftdurchlässigkeiten der eingesetzten Trägermaterialien zum Tragen, die die an sich gegebenen Durchlässigkeiten für Wasserdampf und Luft der Polyurethanhydrogele erhalten, so daß auch die Durchlässigkeit des reinen Trägers erreicht wird. Auch hier ist daher durch Variation z.B. der Foliendicke und -art eine gezielte Steuerung dieser Eigenschaften möglich.

Die erfindungsgemäßen Polyurethangelschäume haben Selbstklebeeigenschaften, insbesondere auf Haut. Sehr stark klebende Schaumgele kleben angenehm auf der Haut und lassen sich schmerzfrei von normaler Haut abziehen.

Für extrem empfindliche Haut sind hingegen schwach klebende Schäume herstellbar, die ein ausreichendes Haftvermögen besitzen, so daß sie haften und leicht fixiert werden können. Hautschäden auch bei extrem empfindlicher Haut werden dabei nicht beobachtet.

Die erfindungsgemäßen Folien können eine hohe Flexibilität aufweisen, da das Schaumgel diese Eigenschaften auch besitzt. Außerdem wird die gute Hautverträglichkeit der Klebegele genutzt. Besonders vorteilhaft werden mikroporöse Polyurethanfolien eingesetzt, da diese Folien besondere Eigenschaften besitzen. Diese Folien sind vorzugsweise keimdicht. Ihre Luft- und Wasserdampfdurchlässigkeit ist variabel. So zeigt z.B. eine Polyurethanfolie mit einer Stärke von etwa 35 um eine Wasserdampf- und Luftdurchlässigkeit von 3500g/m$^2$/24 Stunden. Die Wasserdampfdurchlässigkeit einer 25 μm starken Folie beträgt 6500g/m$^2$/24 Stunden. Eine 50 um starke Folie weist eine Wasserdampfdurchlässigkeit von 2100g/m$^2$/24 Stunden auf. Diese Folientypen sind zudem alle hochflexibel, so daß die hohe Flexibilität der Gelschaummassen gut zur Geltung kommt.

Ein bevorzugter Wundverband gemäß der Erfindung umfaßt eine Polyurethanfolie, die mit einer erfindungsgemäßen Polyurethangelschaum-Schicht versehen ist. Die Dicke dieser Schaumgelschicht kann 0,1 - 10 mm betragen, vorzugsweise jedoch 0,5 - 6 mm und besonders bevorzugt 2 - 4 mm und kann so mit der gewünschten Kapazität abgestimmt werden. Die Schaumdichte kann 0,25 bis 0,7 g/cm$^3$ betragen, bevorzugt 0,3 bis 0,65 g/cm$^3$, insbesondere jedoch 0,45 bis 0,6 g/cm$^3$. Die Flüssigkeitsaufnahme erfolgt sehr rasch. Innerhalb von 90 Minuten werden vorzugsweise 10 bis 60 g Wasser/g Schaumgel, besonders bevorzugt 15 bis 50 g Wasser/g Schaumgel, insbesondere aber 20 bis 40 g Wasser/g Schaumgel aufgenommen. Die Gesamtaufnahmekapazität beträgt vorzugsweise mindestens 20 g Wasser/g Schaumgel, insbesondere mindestens 30 g Wasser/g Schaumgel und beso ers bevorzugt mindestens 40 g Wasser/g Schaumgel. Die Polyurethangelschaumschicht besitzt nur schwache oder sehr geringe Selbstklebeeigenschaften. So kann die Klebkraft bis 0,8 N/cm (gemessen auf Stahl) betragen, bevorzugt 0,06 bis 0,6 N/cm (Stahl) besonders bevorzugt 0,1 bis 0,5 N/cm (Stahl).

Je nach Qualität der eingesetzten Rohstoffe wird hier, bezogen auf die eingesetzte Gewichtsmenge an Polyhydroylverbindungen vorzugsweise 4,7 - 6,9 Gew.-%, bevorzugt 5,0 - 6,4 Gew.-%, insbesondere aber 5,4 - 5,9 Gew.-% an Isocyanat als Vernetzer zugesetzt.

Der Gewichtsanteil an Polyhydroxylverbindungen, bezogen auf das Gesamtgewicht der eingesetzten Rohstoffe, kann zwischen 60 und 80 Gew.-%, bevorzugt zwischen 63 und 77 Gew.-%, insbesondere aber zwischen 65 und 75 Gew.-% liegen.

Der Gewichtsanteil an Superabsorber, bezogen auf das Gesamtgewicht der eingesetzten Rohstoffe, kann zwischen 20 und 40 Gew.-%, bevorzugt zwischen 23 und 37 Gew.-%, insbesondere aber zwischen 25 und 35 Gew.-% betragen.

Dieser Wundverband ist für sehr empfindliche Haut geeignet, wobei die Hautoberfläche in Teilbereichen zerstört sein kann und extrem viel Sekret absondert. Eine deutliche Verteilung der Sekrete über den Wundrand oder Feuchtbereich hinaus findet nicht statt. Eine Fixierung kann beispielsweise zusätzlich mit nichtklebenden Fixierungshilfsmitteln erfolgen.

Besonders wichtig für die Anwendung ist, daß sich das Material in einem Stück entfernen läßt. Aufgrund seiner niedrigen Klebkraft findet es zudem auch bei stark geschädigter Haut ein ideales Einsatzgebiet.

Auch an stark behaarten Körperpartien ist das Material hervorragend einsetzbar, da es sich im wesentlichen ohne Ausreißen der Haare abziehen läßt und sich sogar nahezu schmerzfrei entfernen läßt, da Haare beim Abziehen des Materials nur vorübergehend schwach mitgezogen werden.

Ein weiterer bevorzugter Wundverband gemäß der Erfindung umfaßt eine Polyurethanfolie die mit einer erfindungsgemäßen Polyurethangelschaum-Schicht versehen ist, deren Dicke 0,05 bis 3 mm betragen kann, bevorzugt 0,1 bis 2 mm beträgt, insbesondere jedoch 0,2 bis 1,5 mm beträgt. Die Schaumdichte kann 0,55 bis 0,9 g/cm$^3$ betragen, bevorzugt 0,6 bis 0,85 g/cm$^3$, insbesondere aber 0,65 bis 0,8 g/cm$^3$. Die Flüssigkeitsaufnahme erfolgt ebenfalls rasch. Innerhalb von 90 Min. werden vorzugsweise 5 bis 50 g Wasser/g Schaumgel, besonders bevorzugt 7 bis 40 g Wasser/g Schaumgel und insbesondere 10 bis 30 g Wasser/g Schaumgel aufgenommen. Die Gesamtaufnahmekapazität beträgt vorzugsweise mindestens 15 g Wasser/g Gelschaum, insbesondere mindestens 25 g Wasser/g Schaumgel und besonders bevorzugt mindestens 40 g Wasser/g Schaumgel. Die Polyurethangelschaum-Schicht besitzt gute Selbstklebeeigenschaften. So kann die Klebkraft 0,3 bis 2,5 N/cm (gemessen auf Stahl) betragen, bevorzugt 0,5 bis 2 N/cm (Stahl) und besonders bevorzugt 0,6 bis 1,7 N/cm betragen. Trotz dieser auf Stahl gemessen niedrigen Klebkraftwerte klebt das Material ausgezeichnet auf intakter Haut. Die hervorragenden, zusätzlichen Eigenschaften, die für den schwach klebenden Schaum aufgeführt waren, gelten auch für diese stärker klebende Schaumvariante.

Je nach Qualität der eingesetzten Rohstoffe werden hier, bezogen auf die eingesetzte Gewichtsmenge an Polyhydroxylverbindung vorzugsweise 4,4 - 6,4 Gew.-%, bevorzugt 4,6 - 6,1 Gew.-%, insbesondere aber 5,0 - 5,6 Gew.-% des Isocyanats zugesetzt.

Der Gewichtsanteil an Polyhydroxylverbindungen, bezogen auf das Gesamtgewicht der eingesetzten Rohstoffe, kann zwischen 60 und 80 Gew.-%, bevorzugt zwischen 63 und 77 Gew.-%, insbesondere aber zwischen 65 und 75 Gew.-% liegen.

Der Gewichtsanteil an Superabsorber, bezogen auf das Gesamtgewicht der eingesetzten Rohstoffe, kann zwischen 20 und 40 Gew.-%, bevorzugt zwischen 23 und 37 Gew,-%, insbesondere aber zwischen 25 und 35 Gew.-% betragen.

Der Wundverband haftet gut im Bereich der Gelenke, insbesondere der Hand, da er sehr flexibel ist.

Ein dritter bevorzugter Wundverband entspricht dem vorstehend beschriebenen, stärker selbstklebenden Verband, besitzt aber vorzugsweise eine Schaumgelschicht mit einer Dicke von 0,01 bis 1 mm, insbesondere 0,05 bis 0,7 mm und besonders bevorzugt 0,1 bis 0,5 mm. Die übrigen Merkmale und Eigenschaften entsprechen den vorstehenden Angaben für den stärker selbstklebenden Verband. Der besonders dünne und flexible Wundverband dient dazu, das Verschmieren austretender Restmengen von Wundsekreten zu verhindern und er hat damit z.B. die Funktion einer Schutzabdeckung. Da diese Abdeckung wasserdicht ist, macht auch ein kurzzeitiger Kontakt mit Wasser keine Probleme.

Bevorzugt wird ein Wundverband, der dadurch gekennzeichnet ist, daß

- die Dicke der Schaumgelschicht 0,1 - 10 mm beträgt
- die Schaumdichte 0,25 bis 0,7 g/cm$^3$ beträgt,
- die Wasseraufnahme in 90 min 10 - 60 g Wasser / g Schaumgel beträgt,
- die Gesamtaufnahmekapazität mindestens 20 g / Wasser/g Schaumgel beträgt und
- die Klebkraft bis 0,8 N/cm (gemessen auf Stahl),

beträgt.

Bevorzugt wird auch ein Wundverband, der dadurch gekennzeichnet ist, daß

- die Dicke der Schaumgelschicht 0,05 - 3 mm beträgt,
- die Schaumdichte 0,55 bis 0,9 g/cm$^3$ beträgt,
- die Wasseraufnahme in 90 min 5 - 50 g Wasser / g Schaumgel beträgt,
- die Gesamtaufnahmekapazität mindestens 15 g Wasser / g Schaumgel beträgt und
- die Klebkraft bis 0,3 - 2,5 N/cm (gemessen auf Stahl),

beträgt.

Bevorzugt wird weiterhin ein Wundverband, der dadurch gekennzeichnet ist, daß

- die Dicke der Schaumgelschicht 0,01 - 1 mm beträgt,
- die Schaumdichte 0,55 bis 0,9 g/cm$^3$ beträgt,
- die Wasseraufnahme in 90 min 5 - 50 g Wasser / g Schaumgel beträgt,
- die Gesamtaufnahmekapazität mindestens 15 g Wasser / g Schaumgel beträgt und
- die Klebkraft bis 0,3 - 2,5 N/cm (gemessen auf Strahl),

beträgt.

Für die drei vorstehenden Wundverbände werden Polyurethanfolie mit Dicken von 10 - 100 µm bevorzugt.

Als erfindungsgemäße Träger des Polyurethanschaumgels sind wasserdichte, dabei aber wasserdampfdurchlässige, flexible Polyurethanfolien besonders bevorzugt. Dies gilt insbesondere für die vorstehend beschriebenen bevorzugten Wundverbände.

Derartige Folien können z.B. hergestellt sein aus Polyesterpolyolen und/oder Polyetherpolyolen und/oder anderen Polyolverbindungen mit einer Mindesfunktionalität von 2, die mit aliphatischen oder aromatischen Polyisocyanaten in reiner oder präpolymerisierter Form und einer Mindestfunktionalität von 2 vernetzt sind.

Solche Folien haben vorzugsweise Dicken von 5 - 200 µm, bevorzugt 10 bis 100 µm, besonders bevorzugt 15 bis 70 µm. Die Werte für die Wasserdampfdurchlässigkeit betragen vorzugsweise 500 bis 10 000 g/m$^2$/24h, bevorzugt 700 bis 7000 g/m$^2$/24h, insbesondere aber 1000 bis 5000 g/m$^2$/24h.

Gegenstand der Erfindung ist ferner ein Verfahren zur Herstellung der selbsthaftenden Flächengebilde auf Basis von mit Polyurethan-Gelschäumen beschichteten Trägern. Das Verfahren ist dadurch gekennzeichnet, daß man die oben definierten Schäume oder zur Schaumgelbildung befähigten Reaktionsgemische auf die Oberfläche eines Trägermaterials aufbringt, z.B. nach Direktverfahren oder Umkehrverfahren durch Gießen oder Rakeln, wobei die Oberfläche mit dem gelbildenden Reaktionsgemisch gegebenenfalls nur teilweise bedeckt wird. Die Schichtdicke des Gelschaumes kann z.B. 0,01 mm bis 150 mm betragen, je nachdem, - wie zuvor beschrieben - welche Saugkapazität pro Flächeneinheit erwünscht ist.

Die Herstellung der erfindungsgemäßen Flächengebilde kann kontinuierlich oder diskontinuierlich erfolgen. Die Arbeitsweise hängt von den vorgegebenen, mit einer Haftschicht zu versehenden Flächengebilden ab Wenn man bereits zugeschnittene Trägermaterialien vorliegen hat, ist oftmals eine diskontinuierliche Arbeitsweise vorteilhaft. Die kontinuierliche Arbeitsweise empfiehlt sich bei der Beschichtung von Trägermaterialien, die in endloser Form, z.B. als Rollenware, vorliegen. Der Auftrag der Schaum-Haftschicht auf das Trägermaterial kann dabei direkt oder nach dem Umkehrverfahren erfolgen. Das Reaktionsgemisch läßt sich bei den genannten Verfahren auch, bevor es durch die Reaktion erstarrt, rakeln.

Obwohl die Kohäsion der erfindungsgemäßen Schäume gut ist, kann eine Nachbehandlung der erfindungsgemäßen Schäume und Flächengebilde durch die Bestrahlung mit gamma-Strahlen, wodurch die Kohäsion in der Gelschicht verbessert werden kann, vorteilhaft sein.

Obwohl es, wie erwähnt, möglich ist, die Hafteigenschaften der erfindungsgemäßen Schäume zu steuern, so daß die erhaltenen Schäume oder die damit beschichteten Träger gut selbstklebend sein können, können sie vorteilhaft auf einer der beiden oder auch beiden Großflächen mit einer üblichen Selbstklebemasse beschichtet werden.

Gegenstand der Erfindung ist auch die Verwendung der erfindungsgemäßen Polyurethangelschäume oder der damit hergestellten selbsthaftenden Flächengebilde mit Trägern aus Polyurethanfolien als Wundverbände.

Sie können beispielsweise dazu dienen, Oberflächenwunden oder chirurgische Wunden zu versorgen. Außerdem sind sie als Blasenpflaster geeignet. Sie schützen durch ihre Polsterwirkung die intakte Hautblase, z.B. bei Verbrennungen, und decken die Wundfläche ab, wenn sich die Blase geöffnet hat.

Im Rahmen der vorliegenden Anmeldung sind, soweit nicht anders angegeben, alle Mengen und Prozentangaben auf das Gewicht und die Gesamtzusammensetzung der Zubereitung bezogen.

Die nachfolgenden Beispiele sollen die Erfindung erläutern, ohne daß aber beabsichtigt ist, die Erfindung auf diese Beispiele zu beschränken.

Beispiele

Die folgenden Reaktionskomponenten werden in einem kommerziell verfügbaren Schaummischkopf mit kommerziell verfügbaren Dosiereinrichtungen zusammengeführt und in diesem innig vermischt. Dann wird die Masse auf ein silikonisiertes Papier gestrichen und mit einem weiteren silikonisierten Papier eingedeckt. Anschließend läßt man die Masse bei Raumtemperatur oder bei 80°C ausreagieren. Zur Untersuchung der Eigenschaften werden beide Trennpapiere entfernt.

| Beispiel 1 | |
| --- | --- |
| Polyetherpolyol (Levagel VP SN 100) | 100 kg |
| Superabsorber (Favor SAB 922-SK) | 39 kg |
| Dibutylzinndilaurat | 0.01 kg |
| Diisocyanat (Desmodur PF) | 6.6 kg |
| Stickstoff          etwa 300 l | |

Es resultiert ein schwach klebender, offenporiger Schaum mit 0,45 g/cm$^3$ Dichte. Das Material kann nach 90 Minuten etwa 25 g Wasser pro Gramm seines Eigengewichts aufnehmen.

| Beispiel 2 | |
| --- | --- |
| Polyetherpolyol (Levagel VP SN 100) | 100 kg |
| Superabsorber (Favor SAB 922-SK) | 39 kg |
| Dibutylzinndilaurat | 0.01 kg |
| Diisocyanat (Desmodur PF) | 6.6 kg |
| Stickstoff          etwa 270 l | |

Es resultiert ein schwach klebender, offenporiger Schaum mit 0,55 g/cm$^3$ Dichte. Das Material kann nach 90 Minuten etwa 10 g Wasser pro Gramm seines Eigengewichts aufnehmen.

| Beispiel 3 | |
| --- | --- |
| Polyetherpolyol (Levagel VP SN 100) | 100 kg |
| Superabsorber (Favor SAB 922-SK) | 54 kg |
| Dibutylzinndilaurat | 0.08 kg |
| Diisocyanat (Desmodur PF) | 6.6 kg |
| Stickstoff          etwa 300 l | |

Es resultiert ein klebender, offenporiger Schaum mit einer Dichte von 0,53 g/cm$^3$. Das Material hat nach 90 Minuten 115 g Wasser pro Gramm Eigengewicht aufgenommen.

Die Produkte aller Beispiele können auch einseitig mit einer nicht oder nur schwer ablösbaren, flexiblen Folie aus Polyester kaschiert werden, wobei das Material dann etwa 40% seiner Quellfähigkeit (nach 90 Minuten) verliert.

Die Schichtdicke beträgt etwa 2 mm.

Werden in den vorstehenden Beispielen erfindungsgemäß statt Stickstoff andere Gase wie Luft oder Gasgemische oder Kohlendioxid verwendet, so erhält man gleiche Ergebnisse.

Mit den vorstehend beschriebenen Schäumen oder den schaumbeschichteten Trägern erhält man z.B. medizinisch anwendbare Materialien, insbesondere hervorragend saugfähige Wundpflaster und Wundverbände.

| Beispiel 4 | |
| --- | --- |
| Polyetherpolyol (Levagel VP SN 100) | 100 kg |
| Superabsorber (Favor SAB 922-SK) | 39 kg |
| Dibutylzinndilaurat | 0.01 kg |
| Diisocyanat (Desmodur PF) | 6.6 kg |
| Stickstoff, so daß eine Dichte von 0,45 g / cm$^3$ erhalten wird. | |

Hierzu sind etwa 235 - 285 l Stickstoff geeignet. Schwankungen treten auf, da herstellungsbedingt Teilmengen des Stickstoffs ausgasen können.

Es resultiert ein schwach klebender, auf sich selbst haftender, offenporiger Schaum. Das Material kann bei 3 mm Dicke in 90 Minuten etwa 70 g Wasser pro Gramm seines Eigengewichts aufnehmen.

| Beispiel 5 | |
| --- | --- |
| Polyetherpolyol (Levagel VP SN 100) | 100 kg |
| Superabsorber (Favor SAB 922-SK) | 39 kg |
| Dibutylzinndilaurat | 0.01 kg |
| Diisocyanat (Desmodur PF) | 6.6 kg |
| Stickstoff, so daß eine Dichte von 0,5 g/cm$^3$ erhalten wird. | |

Hierzu sind etwa 200 - 250 l Stickstoff geeignet. Schwankungen treten auf, da herstellungsbedingt Teilmengen des Stickstoffs ausgasen können.

Es resultiert ein schwach klebender, auf sich selbst haftender, offenporiger Schaum. Das Material kann nach 90 Minuten etwa 40 g Wasser pro Gramm seines Eigengewichts aufnehmen.

| Beispiel 6 | |
| --- | --- |
| Polyetherpolyol (Levagel VP SN 100) | 100 kg |
| Superabsorber (Favor SAB 922-SK) | 39 kg |
| Dibutylzinndilaurat | 0.03 kg |
| Diisocyanat (Desmodur PF) | 5,7 kg |
| Stickstoff, so daß eine Dichte von 0,54 g/cm$^3$ erreicht wird. | |

Hierzu sind etwa 165-215 1 Stickstoff geeignet. Schwankungen treten auf, da herstellungsbedingt Teilmengen des Stickstoffs ausgasen können.

Es resultiert ein schwach klebender, gut auf sich selbst haftender offenporiger Schaum. Die Klebkraft auf Stahl wurde mit etwa 0,4 N/cm bestimmt. Die Wasseraufnahme nach 90 Min. beträgt etwa 85 g Wasser/g Probe. Die Schaumdicke beträgt ca. 5mm.

Werden in den vorstehenden Beispielen erfindungsgemäß statt Stickstoff andere Gase wie Luft oder Gasgemische oder Kohlendioxid verwendet, so erhält man gleiche Ergebnisse.

Mit den vorstehend beschriebenen Schäumen und den schaumbeschichteten Trägern erhält man ein hervorragend saugfähiges Schaummaterial, daß für die Aufnahme jeglicher Körperflüssigkeit geeignet ist. Die Produkte sind hervorragend zur Behandlung tiefer Wunden geeignet.

Die folgenden Reaktionskomponenten werden in einem kommerziell verfügbaren Schaummischkopf mit kommerziell verfügbaren Dosiereinrichtungen zusammengeführt und in diesem innig vermischt. Dann wird die Masse auf ein silikonisier

tes Papier gestrichen und mit einer Polyurethanfolie eingedeckt. Anschließend läßt man die Masse bei 80°C ausreagieren. Zur Untersuchung der Eigenschaften wird das Trennpapier entfernt.

| Beispiel 7 | |
|---|---|
| Polyetherpolyol (Levagel VP SN 100) | 100 kg |
| Superabsorber (Favor SAB 922-SK) | 38 kg |
| Dibutylzinndilaurat | 0.02 kg |
| Diisocyanat (Desmodur PF) | 5.7 kg |

Stickstoff, so daß eine Dichte von 0,52 g/cm$^3$ erhalten wird. Hierzu sind etwa 180 - 230 1 Stickstoff geeignet. Schwankungen treten auf, da herstellungsbedingt Teilmengen des Stickstoffs ausgasen können.

Es resultiert ein schwach klebender, offenporiger Schaum. Das Material kann nach 90 Minuten etwa 35 g Wasser pro Gramm seines Eigengewichts aufnehmen. Die Klebkraft auf Stahl beträgt etwa 0,3 N/cm. Die Schichtdicke beträgt ca. 3 mm. Die Flüssigkeit wird im wesentlichen vertikal angesaugt und praktisch nicht zur Seite abgeführt.

Der Schaum kann zusätzlich fixiert werden. Die Wasserdampfdurchlässigkeit beträgt ca. 1/3 der ursprünglichen Wasserdampfdurchlässigkeit von rund 6500 g/m$^2$/24h der Polyurethanfolie. Die Foliendicke beträgt etwa 25 um. Die Folie ist im Handel erhältlich (Rexham PU 77).

| Beispiel 8 | |
|---|---|
| Polyetherpolyol (Levagel VP SN 100) | 100 kg |
| Superabsorber (Favor SAB 922-SK) | 38 kg |
| Dibutylzinndilaurat | 0.02 kg |
| Diisocyanat (Desmodur PF) | 5.3 kg |

Stickstoff, so daß eine Dichte von 0,71 g/cm$^3$ erhalten wird. Hierzu sind etwa 80 - 130 l Stickstoff geeignet. Schwankungen treten auf, da herstellungsbedingt Teilmengen des Stickstoffs ausgasen können.

Es resultiert ein auf Haut gut klebender, offenporiger Schaum. Das Material kann nach 90 Minuten etwa 17 g Wasser pro Gramm seines Eigengewichts aufnehmen. Die Klebkraft auf Stahl beträgt um 1,1 N/cm. Die Schichtdicke beträgt ca. 0,75 mm.

Die Schichtdicke der Polyurethanfolie (Rexham PU 77) liegt bei etwa 25 um und weist eine Wasserdampfdurchlässigkeit von etwa 6500 g/m$^2$/24h auf. Sie reduziert sich durch Aufbringen der Masse um etwa 60%.

| Beispiel 9 | |
|---|---|
| Polyetherpolyol (Levagel VP SN 100) | 100 kg |
| Superabsorber (Favor SAB 922 SK) | 38 kg |
| Dibutylzinndilaurat | 0,02 kg |
| Diisocyanat (Desmodur PF) | 5,3 kg |

Stickstoff, so daß eine Dichte von 0,66 g/cm$^3$ erhalten wird. Hierzu sind etwa 100 bis 150 l Stickstoff geeignet. Schwankungen treten auf, da herstellungsbedingt Teilmengen des Stickstoffs ausgasen können.

Man erhält einen sehr gut auf Haut klebenden Schaum. Die Klebkraft auf Stahl wird zu etwa 0,6 N/cm bestimmt.

Die Wasseraufnahme beträgt ca. 13 g Wasser/g Probe. Es wurde eine Polyurethanfolie von 50 μm Dicke mit einer Wasserdampfdurchlässigkeit von 2100 g/m²/24h verwendet (Rexham 91i). Die Schichtdicke beträgt 0,2 mm.

Werden in den vorstehenden Beispielen erfindungsgemäß statt Stickstoff andere Gase wie Luft oder Gasgemische oder Kohlendioxid verwendet, so erhält man entsprechende Ergebnisse.

Mit den vorstehend beschriebenen Schäumen und den schaumbeschichteten Trägern erhält man hervorragend saugfähige Wundpflaster und Wundverbände.

**Patentansprüche**

1. Selbstklebende, hydrophile Polyurethangelschäume, erhältlich aus

   1. einem Polyurethangel, welches

      (A) 15-62 Gew.-%, vorzugsweise 20-57 Gew.-%, besonders bevorzugt 25-47 Gew.-%, bezogen auf die Summe aus (A) und (B), eines kovalent vernetzten Polyurethans als hochmolekulare Matrix und

      (B) 85-38 Gew.-%, vorzugsweise 80-43 Gew.-%, besonders bevorzugt 75-53 Gew.-%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie gegebenenfalls

      (C) 0-100 Gew.-%, bezogen auf die Summe aus (A) und (B), an Füll- und/oder Zusatzstoffen enthält, und welches erhältlich ist durch Umsetzung einer Mischung von

         a) einem oder mehreren Polyisocyanaten,
         b) einer oder mehreren Polyhydroxylverbindungen mit einem mittleren Molekulargewicht zwischen 1000 und 12000, und einer mittleren OH-Zahl zwischen 20 und 112,
         c) gegebenenfalls Katalysatoren oder Beschleunigern für die Reaktion zwischen Isocyanat- und Hydroxylgruppen sowie gegebenenfalls
         d) aus der Polyurethanchemie an sich bekannten Füllund Zusatzstoffen, wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxylverbindung ($F_P$) zwischen 3 und 6 beträgt und die Isocyanatkennzahl (K) der Formel

$$K = \frac{300 \pm X}{(F_I \cdot F_P) - 1} + 7$$

         gehorcht, in welcher $X \leq 120$, vorzugsweise $X \leq 100$, besonders bevorzugt $X \leq 90$ ist und die Kennzahl K bei Werten zwischen 15 und 70 liegt,

   2. einem Wasser absorbierendem Material und

   3. einem nichtwäßrigem Schäumungsmittel.

2. Polyurethangelschaum gemäß Anspruch 1, dadurch gekennzeichnet, daß das Polyurethangel (A) 20-57 Gew.-%, bezogen auf die Summe aus (A) und (B), des kovalent vernetzten Polyurethans und (B) 80-43 Gew.-%, bezogen auf die Summe aus (A) und (B), der Polyhydroxylverbindungen des flüssigen Dispersionsmittels enthält.

3. Polyurethangelschaum gemäß Anspruch 1, gedurch gekennzeichnet, daß das Polyurethangel (A) 25-47 Gew.-% des kovalent vernetzten Polyurethans und (B) 75-53 Gew.-% der Polyhydroxylverbindungen des flüssigen Dispersionsmittels enthält.

4. Polyurethangelschaum gemäß Anspruch 1, dadurch gekennzeichnet, daß die Polyhydroxylverbindungen Polyetherpolyole sind.

5. Polyurethangelschäume nach Anspruch 1, dadurch gekennzeichnet, daß die Diisocyanate gewählt werden aus der Gruppe der unmodifizierten aromatischen oder aliphatischen Diisocyanate, beispielsweise 4,4'-Diisocyanatodiphenylmethan, und der durch Präpolymerisierung mit Polyolen oder Polyetherpolyolen gebildeten, modifizierten Produkte, beispielsweise des durch Präpolymerisierung mit Tripropylenglycol verflüssigten 4,4'-Diisocyanatodiphenylmethans.

6. Polyurethangelschäume nach Anspruch 1, dadurch gekennzeichnet, daß die Beschleuniger gewählt werden aus der Gruppe bestehend aus

- Organischen Säuren, insbesondere
  p-Toluolsulfonsäure,
  n-Butylphosphorsäure
- Organozinnverbindungen, einschließlich deren Salze organischer und anorganischer Säuren, insbesondere Zinnnaphthenat, Zinnbenzoat, Dibutylzinndioctoat, Dibutylzinndiacetat, Zinn-II-ethylhexoat und Dibutylzinndiacetat
- Eisensalzen höherer Fettsäuren, insbesondere Eisenstearat
- Aminen, zum Beispiel Isophorondiamin, Methylendianilin, Imidazole
- tertiären Aminen, insbesondere Trialkylamine, wobei die Alkylreste jeweils vorteilhaft 2 - 6 Kohlenstoffatome haben.

7. Polyurethangelschaum gemäß Anspruch 1, dadurch gekennzeichnet, daß das Wasser absorbierende Material ein Superabsorber ist.

8. Polyurethangelschaum gemäß Anspruch 1, dadurch gekennzeichnet, daß die nichtwäßrigen Schäumungsmittel Stickstoff, Luft oder Kohlendioxid oder Gemische dieser Gase sind.

9. Polyurethangelschäume gemäß Anspruch 1, erhältlich aus

| 20 | 95 | Gew.-% Polyhydroxylverbindung |
|---|---|---|
| 1 | 60 | Gew.-% Polyisocyanat |
| 5 | 60 | Gew.-% Superabsorber |
| 0.0001 | 10 | Gew.-% Beschleuniger |

und dem nichtwäßrigen Schäumungsmittel in einer Menge, die Dichten von 0,15 bis 1,1 g/cm$^3$ ergibt.

10. Polyurethangelschäume gemäß Anspruch 1, erhältlich aus

| 50 | 70 | Gew.-% Polyhydroxylverbindung |
|---|---|---|
| 2 | 25 | Gew.-% Polyisocyanat |
| 5 | 40 | Gew.-% Superabsorber |
| 0.001 | 1 | Gew.-% Beschleuniger |

und dem nichtwäßrigen Schäumungsmittel in einer Menge, die Dichten von 0,3 bis 1,0 g/cm$^3$ ergibt.

11. Verfahren zur Herstellung der Polyurethanschäume gemäß Anspruch 1, dadurch gekennzeichnet, daß man

1. die vorstehend unter 1. genannten und definierten Komponenten (A), (B) und (C) des Polyurethangeles

2. ein Wasser absorbierendes Material und

3. ein nichtwäßriges Schäumungsmittel

vereinigt und miteinander mischt und schäumt.

**12.** Selbsthaftende Flächengebilde bestehend aus einem flächigen Träger mit einem Auftrag aus einem Polyurethanschaumgel gemäß Anspruch 1.

**13.** Verfahren zur Herstellung von Flächengebilden gemäß Anspruch 12, dadurch gekennzeichnet, daß man auf ein Trägermaterial einen Polyurethanschaum oder ein zur Schaumbildung befähigtes Gemisch gemäß Anspruch 1 aufbringt.

**14.** Verwendung der erfindungsgemäßen Polyurethangelschäume gemäß Anspruch 1 oder der damit hergestellten selbsthaftenden Flächengebilde zur Herstellung von Mitteln für die medizinische Behandlung von Defektwunden bzw. zu deren Prophylaxe, Heftpflaster oder Wundverband oder Wundpflaster, Bandage oder Binde sowie als Schutz und Polstermaterial, insbesondere von Mitteln zur Prophylaxe.

**15.** Verwendung der Polyurethangelschäume gemäß Anspruch 1 zur Herstellung von Flächengebilden für die Versorgung von Wunden.

**16.** Hydrophile Polyurethangelschäume, erhältlich aus

1. einem Polyurethangel, welches

   (A) 35-62 Gew.-%, vorzugsweise 42-60 Gew.-%, besonders bevorzugt 49-57 Gew.-%, bezogen auf die Summe aus (A) und (B), eines kovalent vernetzten Polyurethans als hochmolekulare Matrix und

   (B) 65-38 Gew.-%, vorzugsweise 58-40 Gew.-%, besonders bevorzugt 51-43 Gew.-%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie gegebenenfalls

   (C) 0-100 Gew.-%, bezogen auf die Summe aus (A) und (B), an Füll- und/oder Zusatzstoffen enthält, und welches erhältlich ist durch Umsetzung einer Mischung von

   a) einem oder mehreren Polyisocyanaten,
   b) einer oder mehreren Polyhydroxylverbindungen mit einem mittleren Molekulargewicht zwischen 1000 und 12000, und einer mittleren OH-Zahl zwischen 20 und 112,
   c) gegebenenfalls Katalysatoren oder Beschleunigern für die Reaktion zwischen Isocyanat- und Hydroxylgruppen sowie gegebenenfalls
   d) aus der Polyurethanchemie an sich bekannten Füll- und Zusatzstoffen,

   wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxylverbindung ($F_P$) zwischen 3 und 6 beträgt und die Isocyanatkennzahl (K) der Formel

$$K = \frac{300 \pm X}{(F_I \cdot F_P) - 1} + 7$$

   gehorcht, in welcher $X \leq 120$, vorzugsweise $X \leq 100$, besonders bevorzugt $X \leq 90$ ist und die Kennzahl K bei Werten zwischen 15 und 70 liegt, wobei die angegebenen Mittelwerte von Molekulargewicht und OH-Zahl als Zahlenmittel zu verstehen sind,

2. einem Wasser absorbierendem Material und

3. einem nichtwäßrigem Schäumungsmittel.

**17.** Polyurethangelschaum gemäß Anspruch 16, dadurch gekennzeichnet, daß das Polyurethangel

(A) 42-60 Gew.-%, bezogen auf die Summe aus (A) und (B),
des kovalent vernetzten Polyurethans
und
(B) 58-40 Gew.-%, bezogen auf die Summe aus (A) und (B),
der Polyhydroxylverbindungen des flüssigen Dispersionsmittels enthält.

18. Polyurethangelschaum gemäß Anspruch 16, dadurch gekennzeichnet, daß das Polyurethangel

(A) 49-57 Gew.-%, bezogen auf die Summe aus (A) und (B),
des kovalent vernetzten Polyurethans
und

(B) 51-43 Gew.-% bezogen auf die Summe (A) und (B), der Polyhydroxylverbindungen als flüssiges Dispersionsmittels enthält.

19. Polyurethangelschaum gemäß Anspruch 16, dadurch gekennzeichnet, daß die Polyhydroxylverbindungen Polyetherpolyole sind.

20. Polyurethangelschäume nach Anspruch 16, dadurch gekennzeichnet, daß die Diisocyanate gewählt werden aus der Gruppe der unmodifizierten aromatischen oder aliphatischen Diisocyanate, beispielsweise 4,4'-Diisocyanatodiphenylmethan, und der durch Präpolymerisierung mit Polyolen oder Polyetherpolyolen gebildeten, modifizierten Produkte, beispielsweise des durch Präpolymerisierung mit Tripropylenglycol verflüssigten 4,4'-Diisocyanatodiphenylmethans.

21. Polyurethangelschäume nach Anspruch 16, dadurch gekennzeichnet, daß die Beschleuniger gewählt werden aus der Gruppe bestehend aus

- Organischen Säuren, insbesondere
  p-Toluolsulfonsäure,
  n-Butylphosphorsäure
- Organozinnverbindungen, einschließlich deren Salze organischer und anorganischer Säuren, insbesondere Zinnnaphthenat, Zinnbenzoat, Dibutylzinndioctoat, Dibutylzinndiacetat, Zinn-II-ethylhexoat und Dibutylzinndiethylhexoat
- Eisensalzen höherer Fettsäuren, insbesondere Eisenstearat
- Aminen, zum Beispiel Isophorondiamin, Methylendianilin, Imidazole
- tertiären Aminen, insbesondere Trialkylamine, wobei die Alkylreste jeweils vorteilhaft 2 - 6 Kohlenstoffatome haben.

22. Polyurethangelschaum gemäß Anspruch 16, dadurch gekennzeichnet, daß das Wasser absorbierende Material ein Superabsorber ist.

23. Polyurethangelschaum gemäß Anspruch 16, dadurch gekennzeichnet, daß die nichtwäßrigen Schäumungsmittel Stickstoff, Luft oder Kohlendioxid oder Gemische dieser Gase sind.

24. Polyurethangelschäume gemäß Anspruch 16, erhältlich aus

| 40 | 95 | Gew.-% Polyhydroxylverbindung |
| 1 | 20 | Gew.-% Polyisocyanat |
| 1 | 60 | Gew.-% Superabsorber |
| 0.0001 | 10 | Gew.-% Beschleuniger |

und dem nichtwäßrigen Schäumungsmittel in einer Menge, die Dichten von 0,15 bis 1,1 g/cm$^3$ ergibt.

25. Polyurethangelschäume gemäß Anspruch 16, erhältlich aus

| 55 | 85 | Gew.-% Polyhydroxylverbindung |

EP 0 665 856 B1

(fortgesetzt)

| 2 | 10 | Gew.-% Polyisocyanat |
| 20 | 40 | Gew.-% Superabsorber |
| 0.001 | 1 | Gew.-% Beschleuniger |

und dem nichtwäßrigen Schäumungsmittel in einer Menge, die Dichten von 0,45 bis 0,6 g/cm$^3$ ergibt.

**26.** Verfahren zur Herstellung der Polyurethanschäume gemäß Anspruch 16, dadurch gekennzeichnet, daß man

1. die vorstehend unter 1. genannten und definierten Komponenten (A), (B) und (C) des Polyurethangeles

2. ein Wasser absorbierendes Material und

3. ein nichtwäßriges Schäumungsmittel

vereinigt und miteinander mischt und schäumt.

**27.** Verwendung der Polyurethangelschäume gemäß Anspruch 16 oder der damit hergestellten Flächengebilde zur Herstellung von Mitteln für die medizinische Behandlung von tiefen Wunden, Defekt- wunden und Wundhöhlen sowie Mitteln zum Binden von Speichel oder zum Trocknen oder Trockenhalten von Zahnhöhlen.

**28.** Polyurethangelschäume gemäß Anspruch 16, dadurch gekennzeichnet, daß die Gewichtsmenge des Wasser absorbierenden Materials 24 bis 67 Gew.-% des Gewichts der Polyhydroxylverbindungen beträgt.

**29.** Polyurethangelschäume gemäß Anspruch 16, dadurch gekennzeichnet, daß die Isocyanat-Gewichtsmenge 4,7

- 6,9 Gew.-% des Gewichts der Polyhydroxylverbindungen beträgt.

**30.** Polyurethangelschaum gemäß Anspruch 16, dadurch gekennzeichnet, daß der Gewichtsanteil der Polyhydroxylverbindungen 60 - 80 Gew.-% bezogen auf das Gesamtgewicht beträgt.

**31.** Wundverbände, umfassend eine Polyurethanfolie als Träger und darauf aufgebrachte hydrophile Polyurethangelschäume, die erhältlich sind aus

1. einem Polyurethangel, welches

(A) 25-62 Gew.-%, vorzugsweise 30-60 Gew.-%, besonders bevorzugt 40-57 Gew.-X, bezogen auf die Summe aus (A) und (B), eines kovalent vernetzten Polyurethans als hochmolekulare Matrix und

(B) 75-38 Gew.-X, vorzugsweise 70-40 Gew.-X, besonders bevorzugt 60-43 Gew.-%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie gegebenenfalls

(C) 0-100 Gew.-%, bezogen auf die Summe aus (A) und (B), an Füll- und/oder Zusatzstoffen enthält, und welches erhältlich ist durch Umsetzung einer Mischung von

a) einem oder mehreren Polyisocyanaten,
b) einer oder mehreren Polyhydroxylverbindungen mit einem mittleren Molekulargewicht zwischen 1000 und 12000, und einer mittleren OH-Zahl zwischen 20 und 112,
c) gegebenenfalls Katalysatoren oder Beschieunigern für die Reaktion zwischen Isocyanatund Hy- droxylgruppen sowie gegebenenfalls

24

d) aus der Polyurethanchemie an sich bekannten Füll- und Zusatzstoffen,

wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxylverbindung ($F_P$) zwischen 3 und 6 beträgt und die Isocyanatkennzahl (K) der Formel

$$K = \frac{300 \pm X}{(F_I \cdot F_P) - 1} + 7$$

gehorcht, in welcher $X \leq 120$, vorzugsweise $X \leq 100$, besonders bevorzugt $X \leq 90$ ist und die Kennzahl K bei Werten zwischen 15 und 70 liegt, wobei die angegebenen Mittelwerte von Molekulargewicht und OH-Zahl als Zahlenmittel zu verstehen sind,

2. einem Wasser absorbierenden Material und

3. einem nichtwäßrigen Schäumungsmittel.

32. Wundverband gemäß Anspruch 31, dadurch gekennzeichnet, daß das Polyurethangel

(A) 30-60 Gew.-%, bezogen auf die Summe aus (A) und (B), des kovalent vernetzten Polyurethans und
(B) 70-40 Gew.-%, bezogen auf die Summe aus (A) und (B), der Polyhydroxylverbindungen als flüssiges Dispersionsmittel enthält.

33. Wundverband gemäß Anspruch 31, dadurch gekennzeichnet, daß das Polyurethangel

(A) 40-57 Gew.-%, bezogen auf die Summe (A) und (B), des kovalent vernetzten Polyurethans und

(B) 30-43 Gew.-%, bezogen auf die Summe (A) und (B), der Polyhydroxylverbindungen als flüssiges Dispersionsmittel enthält.

34. Wundverband gemäß Anspruch 31, dadurch gekennzeichnet, daß die Polyhydroxylverbindungen Polyetherpolyole sind.

35. Wundverband nach Anspruch 31, dadurch gekennzeichnet, daß die Diisocyanate gewählt werden aus der Gruppe der unmodifizierten aromatischen oder aliphatischen Diisocyanate, beispielsweise 4,4'-Diisocyanatodiphenylmethan, und der durch Präpolymerisierung mit Polyolen oder Polyetherpolyolen gebildeten, modifizierten Produkte, beispielsweise des durch Präpolymerisierung mit Tripropylenglycol verflüssigten 4,4'-Diisocyanatodiphenylmethans.

36. Wundverband nach Anspruch 31, dadurch gekennzeichnet, daß die Beschleuniger gewählt werden aus der Gruppe bestehend aus

- Organischen Säuren, insbesondere
  p-Toluolsulfonsäure,
  n-Butylphosphorsäure
- Organozinnverbindungen, einschließlich deren Salze organischer und anorganischer Säuren, insbesondere Zinnnaphthenat, Zinnbenzoat, Dibutylzinndioctoat, Dibutylzinndiacetat, Zinn-II-ethylhexoat und Dibutylzinndiethylhexoat
- Eisensalzen höherer Fettsäuren, insbesondere Eisenstearat
- Aminen, zum Beispiel Isophorondiamin, Methylendianilin, Imidazole
- tertiären Aminen, insbesondere Trialkylamine, wobei die Alkylreste jeweils vorteilhaft 2 - 6 Kohlenstoffatome haben.

37. Wundverband gemäß Anspruch 31, dadurch gekennzeichnet, daß das Wasser absorbierende Material ein Superabsorber ist.

**38.** Wundverband gemäß Anspruch 31, dadurch gekennzeichnet, daß die nichtwäßrigen Schäumungsmittel Stickstoff, Luft oder Kohlendioxid oder Gemische dieser Gase sind.

**39.** Wundverband gemäß Anspruch 31, dadurch gekennzeichnet, daß der Polyurethangelschaum erhältlich ist aus

| 20 | 95 | Gew.-% Polyhydroxylverbindung |
|---|---|---|
| 1 | 30 | Gew.-% Polyisocyanat |
| 1 | 60 | Gew.-% Superabsorber |
| 0.0001 | 10 | Gew.-% Beschleuniger |

und dem nichtwäßrigen Schäumungsmittel in einer Menge, die Dichten von 0,15 bis 1,1 g/cm$^3$ ergibt.

**40.** Wundverband gemäß Anspruch 31, dadurch gekennzeichnet, daß der Polyurethangelschaum erhältlich ist aus

| 55 | 85 | Gew.-% Polyhydroxylverbindung |
|---|---|---|
| 2 | 15 | Gew.-% Polyisocyanat |
| 5 | 40 | Gew.-% Superabsorber |
| 0.001 | 1 | Gew.-% Beschleuniger |

und dem nichtwäßrigen Schäumungsmittel in einer Menge, die Dichten von 0,3 bis 1,0 g/cm$^3$ ergibt, insbesondere Dichten von 0,30 bis 0,65 g/cm$^3$, 0,6 bis 0,9 g/cm$^3$ oder 0,8 bis 1,0 g/cm$^3$.

**41.** Verfahren zur Herstellung der Wundverbände gemäß Anspruch 31, dadurch gekennzeichnet, daß man

1. die vorstehend unter 1. genannten und definierten Komponenten (A), (B) und (C) des Polyurethangeles,

2. ein Wasser absorbierendes Material und

3. ein nichtwäßriges Schäumungsmittel vereinigt und miteinander mischt und schäumt und auf eine Polyurethanfolie aufträgt.

**42.** Verwendung der Wundverbände gemäß Anspruch 31 als Heftpflaster, Wundpflaster oder Blasen pflaster.

**43.** Wundverband gemäß Anspruch 31, dadurch gekennzeichnet, daß die Gewichtsmenge des Wasser absorbierenden Materials 24 - 67 Gewichts-%, bezogen auf das Gewicht der Polyhydroxylverbindung, beträgt.

**44.** Wundverbände gemäß Anspruch 31, dadurch gekennzeichnet, daß die Schaumdichte 0,3 bis 0,65 g/cm$^2$, 0,6 bis 0,9 g/cm$^3$ oder 0,8 bis 1,0 g /cm$^3$ beträgt.

**45.** Wundverband gemäß Anspruch 31, dadurch gekennzeichnet, daß

- die Dicke der Schaumgelschicht 0,1- 10 mm beträgt,
- die Schaumdichte 0,25 bis 0,7 g/cm$^3$ beträgt,
- die Isocyanat menge 4,7 - 6,9 Gew.-%, bezogen auf das Gewicht der Polyhydroxylverbindungen beträgt,
- der Gewichtsanteil der Polyhydroxylverbindungen bezogen auf das Gesamtgewicht 60 - 80 Gew.-% beträgt und
- der Gewichtsanteil an Superabsorber bezogen auf das Gesamtgewicht 20 - 40 Gew.-% beträgt.

**46.** Wundverband gemäß Anspruch 31, dadurch gekennzeichnet, daß

- die Dicke der Schaumgelschicht 0,05 - 3 mm beträgt,
- die Schaumdichte 0,55 bis 0,9 g/cm$^3$ beträgt,
- die Isocyanat menge 4,4 - 6,4 Gew.-%, bezogen auf das Gewicht der Polyhydroxylverbindungen beträgt,
- der Gewichtsanteil der Polyhydroxylverbindungen bezogen auf das Gesamtgewicht 60 - 80 Gew.-% beträgt und
- der Gewichtsanteil an Superabsorber bezogen auf das Gesamtgewicht 20 - 40 Gew.-% beträgt.

**47.** Wundverband gemäß Anspruch 31, dadurch gekennzeichnet, daß

- die Dicke der Schaumgelschicht 0,01 - 1 mm beträgt,
- die Schaumdichte 0,55 bis 0,9 g/cm$^3$ beträgt,
- die Isocyanat menge 4,4 - 6,4 Gew.-%, bezogen auf das Gewicht der Polyhydroxylverbindungen beträgt,
- der Gewichtsanteil der Polyhydroxylverbindungen bezogen auf das Gesamtgewicht 60 - 80 Gew.-% beträgt und
- der Gewichtsanteil an Superabsorber bezogen auf das Gesamtgewicht 20 - 40 Gew.-% beträgt.

**48.** Wundverband gemäß Anspruch 31, dadurch gekennzeichnet, daß eine Seite einer Polyurethanfolie mit einer Schicht eines Polyurethangelschaumes versehen ist.

**Claims**

1.  Self-adhesive, hydrophilic polyurethane gel foams obtainable from

    1. a polyurethane gel which comprises

        (A) 15-62% by weight, preferably 20-57% by weight, particularly preferably 25-47% by weight, based on the total of (A) and (B) of a covalently crosslinked polyurethane as high molecular weight matrix and
        (B) 85-38% by weight, preferably 80-43% by weight, particularly preferably 75-53% by weight, based on the total of (A) and (B), of one or more polyhydroxyl compounds which are firmly bound in the matrix by secondary valence forces and have an average molecular weight between 1000 and 12000, preferably between 1500 and 8000, particularly preferably between 2000 and 6000, and an average OH number between 20 and 112, preferably between 25 and 84, particularly preferably between 28 and 56, as liquid dispersant, the dispersant being essentially free of hydroxyl compounds with a molecular weight below 800, preferably below 1000, particularly preferably below 1500, and, where appropriate,
        (C) 0-100% by weight, based on the total of (A) and (B), of fillers and/or additives, and which is obtainable by reacting a mixture of

            a) one or more polyisocyanates
            b) one or more polyhydroxyl compounds with an average molecular weight between 1000 and 12000, and with an average OH number between 20 and 112,
            c) where appropriate catalysts or accelerators for the reaction between isocyanate groups and hydroxyl groups and, where appropriate,
            d) fillers and additives known per se from polyurethane chemistry,
            this mixture being essentially free of hydroxyl compounds with a molecular weight below 800, the average functionality of the polyisocyanates ($F_I$) being between 2 and 4, the average functionality of the polyhydroxyl compound ($F_P$) being between 3 and 6, and the isocyanate index (K) being given by the formula

$$K = \frac{300 \pm X}{(F_I \cdot F_P) - 1} + 7$$

        in which X ≤ 120, preferably X ≤ 100, particularly preferably X ≤ 90, and the index K has values between 15 and 70,

    2. a water-absorbing material and
    3. a non-aqueous foaming agent.

2.  Polyurethane gel foam according to Claim 1, characterized in that the polyurethane gel contains (A) 20-57% by weight, based on the total of (A) and (B), of the covalently crosslinked polyurethane and (B) 80-43% by weight, based on the total of (A) and (B), of the polyhydroxyl compounds of the liquid dispersant.

3.  Polyurethane gel foam according to Claim 1, characterized in that the polyurethane gel contains (A) 25-47% by weight of the covalently crosslinked polyurethane and (B) 75-53% by weight of the polyhydroxyl compounds of the liquid dispersant.

4. Polyurethane gel foam according to Claim 1, characterized in that the polyhydroxyl compounds are polyether polyols.

5. Polyurethane gel foams according to Claim 1, characterized in that the diisocyanates are chosen from the group of unmodified aromatic or aliphatic diisocyanates, for example 4,4'-diisocyanatodiphenylmethane, and of modified products formed by prepolymerization with polyols or polyether polyols, for example of 4,4'-diisocyanatodiphenylmethane liquified by prepolymerization with tripropylene glycol.

6. Polyurethane gel foams according to Claim 1, characterized in that the accelerators are chosen from the group consisting of

- organic acids, in particular
  p-toluenesulphonic acid,
  n-butylphosphonic acid
- organotin compounds, including their salts with organic and inorganic acids, in particular tin naphthenate, tin benzoate, dibutyltin dioctoate, dibutyltin diacetate, tin(II) ethylhexoate and dibutyltin acetate
- iron salts of higher fatty acids, in particular iron stearate
- amines, for example isophoronediamine, methylenedianiline, imidazoles
- tertiary amines, in particular trialkylamines, the alkyl radicals each advantageously having 2-6 carbon atoms.

7. Polyurethane gel foam according to Claim 1, characterized in that the water-absorbing material is a superabsorbent.

8. Polyurethane gel foam according to Claim 1, characterized in that the non-aqueous foaming agents are nitrogen, air or carbon dioxide or mixtures of these gases.

9. Polyurethane gel foams according to Claim 1, obtainable from

| 20 | 95% | by weight of polyhydroxyl compound |
|---|---|---|
| 1 | 60% | by weight of polyisocyanate |
| 5 | 60% | by weight of superabsorbent |
| 0.0001 | 10% | by weight of accelerator |

and the non-aqueous foaming agent in an amount which affords densities of 0.15 to 1.1 g/cm$^3$.

10. Polyurethane gel foams according to Claim 1, obtainable from

| 50 | 70% | by weight of polyhydroxyl compound |
|---|---|---|
| 2 | 25% | by weight of polyisocyanate |
| 5 | 40% | by weight of superabsorbent |
| 0.001 | 1% | by weight of accelerator |

and the non-aqueous foaming agent in an amount which affords densities of 0.3 to 1.0 g/cm$^3$.

11. Process for the production of the polyurethane foams according to Claim 1, characterized in that

1. the components (A), (B) and (C) of the polyurethane gel which are mentioned and defined above under 1.
2. a water-absorbing material and
3. a non-aqueous foaming agent are combined and mixed together and foamed.

12. Self-adhesive sheet-like structures consisting of a sheet-like backing to which is applied a polyurethane foam gel according to Claim 1.

13. Process for the production of sheet-like structures according to Claim 12, characterized in that a polyurethane foam or a mixture capable of foam formation according to Claim 1 is applied to a backing material.

14. Use of the novel polyurethane gel foams according to Claim 1 or of the self-adhesive sheet-like structures produced therewith for the production of compositions for medical treatment of defect wounds or for the prophylaxis thereof, adhesive plaster or wound dressing or wound plaster, bandage or support, and as protection and padding material, in particular of compositions for prophylaxis.

15. Use of the polyurethane gel foams according to Claim 1 for the production of sheet-like structures for the management of wounds.

16. Hydrophilic polyurethane gel foams obtainable from

1. a polyurethane gel which comprises

(A) 35-62% by weight, preferably 42-60% by weight, particularly preferably 49-57% by weight, based on the total of (A) and (B) of a covalently crosslinked polyurethane as high molecular weight matrix and
(B) 65-38% by weight, preferably 58-40% by weight, particularly preferably 51-43% by weight, based on the total of (A) and (B), of one or more polyhydroxyl compounds which are firmly bound in the matrix by secondary valence forces and have an average molecular weight between 1000 and 12000, preferably between 1500 and 8000, particularly preferably between 2000 and 6000, and an average OH number between 20 and 112, preferably between 25 and 84, particularly preferably between 28 and 56, as liquid dispersant, the dispersant being essentially free of hydroxyl compounds with a molecular weight below 800, preferably below 1000, particularly preferably below 1500, and, where appropriate,
(C) 0-100% by weight, based on the total of (A) and (B), of fillers and/or additives, and which is obtainable by reacting a mixture of

a) one or more polyisocyanates
b) one or more polyhydroxyl compounds with an average molecular weight between 1000 and 12000, and with an average OH number between 20 and 112,
c) where appropriate catalysts or accelerators for the reaction between isocyanate groups and hydroxyl groups and, where appropriate,
d) fillers and additives known per se from polyurethane chemistry,

this mixture being essentially free of hydroxyl compounds with a molecular weight below 800, the average functionality of the polyisocyanates ($F_I$) being between 2 and 4, the average functionality of the polyhydroxyl compound ($F_p$) being between 3 and 6, and the isocyanate index (K) being given by the formula

$$K = \frac{300 \pm X}{(F_I \cdot F_P) - 1} + 7$$

in which $X \leq 120$, preferably $X \leq 100$, particularly preferably $X \leq 90$, and the index K has values between 15 and 70, where the stated averages of molecular weight and OH number are to be understood as number averages,
2. a water-absorbing material and
3. a non-aqueous foaming agent.

17. Polyurethane gel foam according to Claim 16, characterized in that the polyurethane gel contains

(A) 42-60% by weight, based on the total of (A) and (B), of the covalently crosslinked polyurethane and
(B) 58-40% by weight, based on the total of (A) and (B), of the polyhydroxyl compounds of the liquid dispersant.

18. Polyurethane gel foam according to Claim 16, characterized in that the polyurethane gel contains

(A) 49-57% by weight, based on the total of (A) and (B), of the covalently crosslinked polyurethane and
(B) 51-43% by weight, based on the total of (A) and (B), of the polyhydroxyl compounds of the liquid dispersant.

19. Polyurethane gel foam according to Claim 16, characterized in that the polyhydroxyl compounds are polyether polyols.

20. Polyurethane gel foams according to Claim 16, characterized in that the diisocyanates are chosen from the group of unmodified aromatic or aliphatic diisocyanates, for example 4,4'-diisocyanatodiphenylmethane, and of modified products formed by prepolymerization with polyols or polyether polyols, for example of 4,4'-diisocyanatodiphenylmethane liquified by prepolymerization with tripropylene glycol.

21. Polyurethane gel foams according to Claim 16, characterized in that the accelerators are chosen from the group consisting of

- organic acids, in particular
  p-toluenesulphonic acid,
  n-butylphosphonic acid
- organotin compounds, including their salts with organic and inorganic acids, in particular tin naphthenate, tin benzoate, dibutyltin dioctoate, dibutyltin diacetate, tin(II) ethylhexoate and dibutyltin diethylhexoate
- iron salts of higher fatty acids, in particular iron stearate
- amines, for example isophoronediamine, methylenedianiline, imidazoles
- tertiary amines, in particular trialkylamines, the alkyl radicals each advantageously having 2-6 carbon atoms.

22. Polyurethane gel foam according to Claim 16, characterized in that the water-absorbing material is a superabsorbent.

23. Polyurethane gel foam according to Claim 16, characterized in that the non-aqueous foaming agents are nitrogen, air or carbon dioxide or mixtures of these gases.

24. Polyurethane gel foams according to Claim 16, obtainable from

| 40 | 95% | by weight of polyhydroxyl compound |
| 1 | 20% | by weight of polyisocyanate |
| 1 | 60% | by weight of superabsorbent |
| 0.0001 | 10% | by weight of accelerator |

and the non-aqueous foaming agent in an amount which affords densities of 0.15 to 1.1 $g/cm^3$.

25. Polyurethane gel foams according to Claim 16, obtainable from

| 55 | 85% | by weight of polyhydroxyl compound |
| 2 | 10% | by weight of polyisocyanate |
| 20 | 40% | by weight of superabsorbent |
| 0.001 | 1% | by weight of accelerator |

and the non-aqueous foaming agent in an amount which affords densities of 0.45 to 0.6 $g/cm^3$.

26. Process for the production of the polyurethane foams according to Claim 16, characterized in that

1. the components (A), (B) and (C) of the polyurethane gel which are mentioned and defined above under 1.
2. a water-absorbing material and
3. a non-aqueous foaming agent are combined and mixed together and foamed.

27. Use of the polyurethane gel foams according to Claim 16 or of the sheet-like structures produced therewith for the production of compositions for medical treatment of deep wounds, defect wounds and wound cavities, and compositions for binding saliva or for drying tooth cavities or for keeping them dry.

28. Polyurethane gel foams according to Claim 16, characterized in that the amount by weight of the water-absorbing material is 24 to 67% by weight of the weight of the polyhydroxyl compounds.

29. Polyurethane gel foams according to Claim 16, characterized in that the amount by weight of isocyanate is 4.7 to 6.9% by weight of the weight of the polyhydroxyl compounds.

30. Polyurethane gel foam according to Claim 16, characterized in that the content by weight of polyhydroxyl compounds is 60-80% by weight based on the total weight.

31. Wound dressings comprising a polyurethane sheet as backing and hydrophilic polyurethane gel foams applied thereto, which are obtainable from

    1. a polyurethane gel which comprises

        (A) 25-62% by weight, preferably 30-60% by weight, particularly preferably 40-57% by weight, based on the total of (A) and (B) of a covalently crosslinked polyurethane as high molecular weight matrix and
        (B) 75-38% by weight, preferably 70-40% by weight, particularly preferably 60-43% by weight, based on the total of (A) and (B), of one or more polyhydroxyl compounds which are firmly bound in the matrix by secondary valence forces and have an average molecular weight between 1000 and 12000, preferably between 1500 and 8000, particularly preferably between 2000 and 6000, and an average OH number between 20 and 112, preferably between 25 and 84, particularly preferably between 28 and 56, as liquid dispersant, the dispersant being essentially free of hydroxyl compounds with a molecular weight below 800, preferably below 1000, particularly preferably below 1500, and, where appropriate,
        (C) 0-100% by weight, based on the total of (A) and (B), of fillers and/or additives, and which is obtainable by reacting a mixture of

        a) one or more polyisocyanates
        b) one or more polyhydroxyl compounds with an average molecular weight between 1000 and 12000, and with an average OH number between 20 and 112,
        c) where appropriate catalysts or accelerators for the reaction between isocyanate groups and hydroxyl groups and, where appropriate,
        d) fillers and additives known per se from polyurethane chemistry,

    this mixture being essentially free of hydroxyl compounds with a molecular weight below 800, the average functionality of the polyisocyanates $(F_I)$ being between 2 and 4, the average functionality of the polyhydroxyl compound $(F_P)$ being between 3 and 6, and the isocyanate index (K) being given by the formula

$$K = \frac{300 \pm X}{(F_I \cdot F_P) - 1} + 7$$

    in which $X \leq 120$, preferably $X \leq 100$, particularly preferably $X \leq 90$, and the index K has values between 15 and 70, where the stated averages of molecular weight and OH number are to be understood as number averages,
    2. a water-absorbing material and
    3. a non-aqueous foaming agent.

32. Wound dressing according to Claim 31, characterized in that the polyurethane gel contains

    (A) 30-60% by weight, based on the total of (A) and (B), of the covalently crosslinked polyurethane and
    (B) 70-40% by weight, based on the total of (A) and (B), of the polyhydroxyl compounds as liquid dispersant.

33. Wound dressing according to Claim 31, characterized in that the polyurethane gel contains

    (A) 40-57% by weight, based on the total of (A) and (B), of the covalently crosslinked polyurethane and
    (B) 30-43% by weight, based on the total of (A) and (B), of the polyhydroxyl compounds as liquid dispersant.

34. Wound dressing according to Claim 31, characterized in that the polyhydroxyl compounds are polyether polyols.

35. Wound dressing according to Claim 31, characterized in that the diisocyanates are chosen from the group of unmodified aromatic or aliphatic diisocyanates, for example 4,4'-diisocyanatodiphenylmethane, and of modified products formed by prepolymerization with polyols or polyether polyols, for example of 4,4'-diisocyanatodiphenylmethane liquified by prepolymerization with tripropylene glycol.

36. Wound dressing according to Claim 31, characterized in that the accelerators are chosen from the groups consisting of

- organic acids, in particular
  p-toluenesulphonic acid,
  n-butylphosphonic acid
- organotin compounds, including their salts with organic and inorganic acids, in particular tin naphthenate, tin benzoate, dibutyltin dioctoate, dibutyltin diacetate, tin(II) ethylhexoate and dibutyltin diethylhexoate
- iron salts of higher fatty acids, in particular iron stearate
- amines, for example isophoronediamine, methylenedianiline, imidazoles
- tertiary amines, in particular trialkylamines, the alkyl radicals each advantageously having 2-6 carbon atoms.

37. Wound dressing according to Claim 31, characterized in that the water-absorbing material is a superabsorbent.

38. Wound dressing according to Claim 31, characterized in that the non-aqueous foaming agents are nitrogen, air or carbon dioxide or mixtures of these gases.

39. Wound dressing according to Claim 31, characterized in that the polyurethane gel foam is obtainable from

| 20 | 95% | by weight of polyhydroxyl compound |
|---|---|---|
| 1 | 30% | by weight of polyisocyanate |
| 1 | 60% | by weight of superabsorbent |
| 0.0001 | 10% | by weight of accelerator |

and the non-aqueous foaming agent in an amount which affords densities of 0.15 to 1.1 $g/cm^3$.

40. Wound dressing according to Claim 31, characterized in that the polyurethane gel foam is obtainable from

| 55 | 85% | by weight of polyhydroxyl compound |
|---|---|---|
| 2 | 15% | by weight of polyisocyanate |
| 5 | 40% | by weight of superabsorbent |
| 0.001 | 1% | by weight of accelerator |

and the non-aqueous foaming agent in an amount which affords densities of 0.3 to 1.0 $g/cm^3$, in particular densities of 0.30 to 0.65 $g/cm^3$, 0.6 to 0.9 $g/cm^3$ or 0.8 to 1.0 $g/cm^3$.

41. Process for the production of the wound dressings according to Claim 31, characterized in that

1. the components (A), (B) and (C) of the polyurethane gel which are mentioned and defined above under 1.
2. a water-absorbing material and
3. a non-aqueous foaming agent are combined and mixed together and foamed and applied to a polyurethane sheet.

42. Use of the wound dressings according to Claim 31, as adhesive plaster, wound plaster or blister plaster.

43. Wound dressing according to Claim 31, characterized in that the amount by weight of the water-absorbing material is 24-67% by weight, based on the weight of the polyhydroxyl compound.

44. Wound dressings according to Claim 31, characterized in that the foam density is 0.3 to 0.65 $g/cm^3$, 0.6 to 0.9 $g/cm^3$ or 0.8 to 1.0 $g/cm^3$.

45. Wound dressing according to Claim 31, characterized in that

-   the thickness of the foam gel layer is 0.1-10 mm,
-   the foam density is 0.25 to 0.7 g/cm$^3$,
-   the amount of isocyanate is 4.7 - 6.9% by weight based on the weight of the polyhydroxyl compounds,
-   the content by weight of polyhydroxyl compounds is 60-80% by weight based on the total weight and
-   the content by weight of superabsorbent is 20-40% by weight based on the total weight.

**46.** Wound dressing according to Claim 31, characterized in that

-   the thickness of the foam gel layer is 0.05-3 mm,
-   the foam density is 0.55 to 0.9 g/cm$^3$,
-   the amount of isocyanate is 4.4 - 6.4% by weight based on the weight of the polyhydroxyl compounds,
-   the content by weight of polyhydroxyl compounds is 60-80% by weight based on the total weight and
-   the content by weight of superabsorbent is 20-40% by weight based on the total weight.

**47.** Wound dressing according to Claim 31, characterized in that

-   the thickness of the foam gel layer is 0.01-1 mm,
-   the foam density is 0.55 to 0.9 g/cm$^3$,
-   the amount of isocyanate is 4.4 - 6.4% by weight based on the weight of the polyhydroxyl compounds,
-   the content by weight of polyhydroxyl compounds is 60-80% by weight based on the total weight and
-   the content by weight of superabsorbent is 20-40% by weight based on the total weight.

**48.** Wound dressing according to Claim 31, characterized in that one side of a polyurethane sheet is provided with a layer of a polyurethane gel foam.

**Revendications**

**1.** Mousses de gels de polyuréthanne hydrophiles autocollantes pouvant être obtenues à partir de

1. un gel de polyuréthanne qui contient

(A) 15-62% en poids, de préférence 20-57% en poids, particulièrement préférablement 25-47% en poids, par rapport à la somme de (A) et (B), d'un polyuréthanne réticulé par covalence en tant que matrice à poids moléculaire élevé et
(B) 85-38% en poids, de préférence 80-43% en poids, particulièrement préférablement 75-53% en poids, par rapport à la somme de (A) et (B), d'un ou de plusieurs composés polyhydroxylés liés fermement dans la matrice par des forces de valence secondaires et ayant un poids moléculaire moyen compris entre 1 000 et 12 000, de préférence entre 1 500 et 8 000, particulièrement préférablement entre 2 000 et 6 000, et un indice d'OH moyen compris entre 20 et 112, de préférence entre 25 et 84, particulièrement préférablement entre 28 et 56, en tant qu'agent dispersant liquide, l'agent dispersant étant essentiellement exempt de composés hydroxylés ayant un poids moléculaire inférieur à 800, de préférence inférieur à 1 000, particulièrement préférablement inférieur à 1 500, et éventuellement
(C) 0-100% en poids, par rapport à la somme de (A) et (B), de charges et/ou d'additifs, et qui peut être obtenu par réaction d'un mélange de

a) un ou plusieurs polyisocyanates
b) un ou plusieurs composés polyhydroxylés ayant un poids moléculaire moyen compris entre 1 000 et 12 000, et un indice d'OH moyen compris entre 20 et 112,
c) éventuellement des catalyseurs ou des accélérateurs pour la réaction entre les groupes isocyanate et hydroxyle, et éventuellement
d) des charges et additifs connus en soi par la chimie des polyuréthannes, ce mélange étant essentiellement exempt de composés hydroxylés ayant un poids moléculaire inférieur à 800, la fonctionnalité moyenne des polyisocyanates ($F_I$) étant comprise entre 2 et 4, la fonctionnalité moyenne du composé polyhydroxylé ($F_P$) étant comprise entre 3 et 6 et l'indice d'isocyanate (K) obéissant à la formule

$$K = \frac{300 \pm X}{(F_I \cdot F_P) - 1} + 7$$

dans laquelle X ≤ 120, de préférence X ≤ 100, particulièrement préférablement X ≤ 90 et l'indice K vaut entre 15 et 70,

2. un matériau absorbant l'eau et
3. un agent moussant non aqueux.

2. Mousse de gel de polyuréthanne selon la revendication 1, caractérisée en ce que le gel de polyuréthanne contient (A) 20-57% en poids, par rapport à la somme de (A) et (B), du polyuréthanne réticulé par covalence et (B) 80-43% en poids, par rapport à la somme de (A) et (B), des composés polyhydroxylés de l'agent dispersant liquide.

3. Mousse de gel de polyuréthanne selon la revendication 1, caractérisée en ce que le gel de polyuréthanne contient (A) 25-47% en poids du polyuréthanne réticulé par covalence et (B) 75-53% en poids des composés polyhydroxylés de l'agent dispersant liquide.

4. Mousse de gel de polyuréthanne selon la revendication 1, caractérisée en ce que les composés polyhydroxylés sont des polyéther-polyols.

5. Mousses de gels de polyuréthanne selon la revendication 1, caractérisées en ce que les diisocyanates sont choisis parmi le groupe constitué par les diisocyanates aliphatiques ou aromatiques non modifiés, par exemple le 4,4'-diisocyanatodiphénylméthane, et les produits modifiés formés par prépolymérisation avec des polyols ou des polyéther-polyols, par exemple du 4,4'-diisocyanatodiphénylméthane liquéfié par prépolymérisation avec du tripropylèneglycol.

6. Mousses de gels de polyuréthanne selon la revendication 1, caractérisées en ce que les accélérateurs sont choisis parmi le groupe constitué par

- des acides organiques, en particulier l'acide p-toluènesulfonique, l'acide n-butylphosphorique
- des composés organoétain, y compris leurs sels avec des acides organiques et inorganiques, en particulier le naphténate d'étain, le benzoate d'étain, le dioctoate de dibutylétain, le diacétate de dibutylétain, l'éthylhexoate d'étain II et le diacétate de dibutylétain
- des sels de fer d'acides gras supérieurs, en particulier le stéarate de fer
- des amines, par exemple l'isophoronediamine, la méthylènedianiline, des imidazoles
- des amines tertiaires, en particulier des trialkylamines, les radicaux alkyle ayant chacun avantageusement 2-6 atomes de carbone.

7. Mousse de gel de polyuréthanne selon la revendication 1, caractérisée en ce que le matériau absorbant l'eau est un superabsorbant.

8. Mousse de gel de polyuréthanne selon la revendication 1, caractérisée en ce que les agents moussants non aqueux sont l'azote, l'air ou le dioxyde de carbone ou des mélanges de ces gaz.

9. Mousses de gels de polyuréthanne selon la revendication 1, pouvant être obtenues à partir de

20-95% en poids de composé polyhydroxylé
1-60% en poids de polyisocyanate
5-60% en poids de superabsorbant
0,0001-10% en poids d'accélérateur

et de l'agent moussant non aqueux en une quantité qui donne lieu à des densités de 0,15 à 1,1 g/cm³.

10. Mousses de gels de polyuréthanne selon la revendication 1, pouvant être obtenues à partir de

50-70% en poids de composé polyhydroxylé
2-25% en poids de polyisocyanate

5-40% en poids de superabsorbant
0,001-1% en poids d'accélérateur

et de l'agent moussant non aqueux en une quantité qui donne lieu à des densités de 0,3 à 1,0 g/cm$^3$.

11. Procédé de préparation des mousses de polyuréthanne selon la revendication 1, caractérisé en ce que

    1. les composants (A), (B) et (C) du gel de polyuréthanne, mentionnés et définis ci-dessus en 1,
    2. un matériau absorbant l'eau et
    3. un agent moussant non aqueux sont combinés, mélangés les uns avec les autres et mis sous forme de mousse.

12. Structures en feuilles autocollantes constituées d'un support mince sur lequel est appliqué un gel de mousse de polyuréthanne selon la revendication 1.

13. Procédé de préparation de structures en feuilles selon la revendication 12, caractérisé en ce que l'on applique une mousse de polyuréthanne ou un mélange capable de former une mousse selon la revendication 1 sur un matériau de support.

14. Utilisation des mousses de gel de polyuréthanne selon l'invention selon la revendication 1 ou des structures en feuilles autocollantes préparées avec celles-ci, pour la préparation de produits pour le traitement médical de blessures défectueuses ou pour leur prophylaxie, en tant que sparadrap, pansement ou emplâtre vulnéraire, bandage ou bande et en tant que protection et matériau de rembourrage, en particulier de produits pour la prophylaxie.

15. Utilisation des mousses de gels de polyuréthanne selon la revendication 1, pour la préparation de structures en feuilles pour le soin des blessures.

16. Mousses de gels de polyuréthanne hydrophiles pouvant être obtenues à partir de

    1. un gel de polyuréthanne qui contient

        (A) 35-62% e poids, de préférence 40-60% en poids, particulièrement préférablement 49-57% en poids, par rapport à la somme de (A) et (B), d'un polyuréthanne réticulé par covalence en tant que matrice à poids moléculaire élevé et
        (B) 65-38% en poids, de préférence 58-40% en poids, particulièrement préférablement 51-43% en poids, par rapport à la somme de (A) et (B), d'un ou de plusieurs composés polyhydroxylés liés fermement dans la matrice par des forces de valence secondaires et ayant un poids moléculaire moyen compris entre 1 000 et 12 000, de préférence entre 1 500 et 8 000, particulièrement préférablement entre 2 000 et 6 000, et un indice d'OH moyen compris entre 20 et 112, de préférence entre 25 et 84, particulièrement préférablement entre 28 et 56, en tant qu'agent dispersant liquide, l'agent dispersant étant essentiellement exempt de composés hydroxylés ayant un poids moléculaire inférieur à 800, de préférence inférieur à 1 000, particulièrement préférablement inférieur à 1 500, et éventuellement
        (C) 0-100% en poids, par rapport à la somme de (A) et (B), de charges et/ou d'additifs, et qui peut être obtenu par réaction d'un mélange de

            a) un ou plusieurs polyisocyanates
            b) un ou plusieurs composés polyhydroxylés ayant un poids moléculaire moyen compris entre 1 000 et 12 000, et un indice d'OH moyen compris entre 20 et 112,
            c) éventuellement des catalyseurs ou des accélérateurs pour la réaction entre les groupes isocyanate et hydroxyle, et éventuellement
            d) des charges et additifs connus en soi par la chimie des polyuréthannes, ce mélange étant essentiellement exempt de composés hydroxylés ayant un poids moléculaire inférieur à 800, la fonctionnalité moyenne des polyisocyanates ($F_I$) étant comprise entre 2 et 4, la fonctionnalité moyenne du composé polyhydroxylé ($F_P$) étant comprise entre 3 et 6 et l'indice d'isocyanate (K) obéissant à la formule

$$K = \frac{300 \pm X}{(F_I \cdot F_P) - 1} + 7$$

dans laquelle $X \leq 120$, de préférence $X \leq 100$, particulièrement préférablement $X \leq 90$ et l'indice K vaut entre 15 et 70, où les valeurs moyennes indiquées du poids moléculaire et de l'indice d'OH doivent être comprises comme des moyennes en nombre,

2. un matériau absorbant l'eau et

3. un agent moussant non aqueux.

17. Mousse de gel de polyuréthanne selon la revendication 16, caractérisée en ce que le gel de polyuréthanne contient

(A) 42-60% en poids, par rapport à la somme de (A) et(B), du polyuréthanne réticulé par covalence et
(B) 58-40% en poids, par rapport à la somme de (A) et (B), des composés polyhydroxylés de l'agent dispersant liquide.

18. Mousse de gel de polyuréthanne selon la revendication 16, caractérisée en ce que le gel de polyuréthanne contient

(A) 49-57% en poids, par rapport à la somme de (A) et(B), du polyuréthanne réticulé par covalence et
(B) 51-43% en poids, par rapport à la somme de (A) et (B), des composés polyhydroxylés en tant qu'agent dispersant liquide.

19. Mousse de gel de polyuréthanne selon la revendication 16, caractérisée en ce que les composés polyhydroxylés sont des polyéther-polyols.

20. Mousses de gels de polyuréthanne selon la revendication 16, caractérisées en ce que les diisocyanates sont choisis parmi le groupe constitué par les diisocyanates aliphatiques ou aromatiques non modifiés, par exemple le 4,4'-diisocyanatodiphénylméthane, et les produits modifiés formés par prépolymérisation avec des polyols ou des polyéther-polyols, par exemple du 4,4'-diisocyanatodiphénylméthane liquéfié par prépolymérisation avec du tripropylèneglycol.

21. Mousses de gels de polyuréthanne selon la revendication 16, caractérisées en ce que les accélérateurs sont choisis parmi le groupe constitué par

- des acides organiques, en particulier l'acide p-toluènesulfonique, l'acide n-butylphosphorique
- des composés organoétain, y compris leurs sels avec des acides organiques et inorganiques, en particulier le naphténate d'étain, le benzoate d'étain, le dioctoate de dibutylétain, le diacétate de dibutylétain, l'éthylhexoate d'étain II et le diéthylhexoate de dibutylétain
- des sels de fer d'acides gras supérieurs, en particulier le stéarate de fer
- des amines, par exemple l'isophoronediamine, la méthylènedianiline, des imidazoles
- des amines tertiaires, en particulier des trialkylamines, les radicaux alkyle ayant chacun avantageusement 2-6 atomes de carbone.

22. Mousse de gel de polyuréthanne selon la revendication 16, caractérisée en ce que le matériau absorbant l'eau est un superabsorbant.

23. Mousse de gel de polyuréthanne selon la revendication 16, caractérisée en ce que les agents moussants non aqueux sont l'azote, l'air ou le dioxyde de carbone ou des mélanges de ces gaz.

24. Mousses de gels de polyuréthanne selon la revendication 16, pouvant être obtenues à partir de

40-95% en poids de composé polyhydroxylé
1-20% en poids de polyisocyanate
1-60% en poids de superabsorbant
0,0001-10% en poids d'accélérateur

et de l'agent moussant non aqueux en une quantité qui donne lieu à des densités de 0,15 à 1,1 g/cm$^3$.

**25.** Mousses de gels de polyuréthanne selon la revendication 16, pouvant être obtenues à partir de

55-85% en poids de composé polyhydroxylé
2-10% en poids de polyisocyanate
20-40% en poids de superabsorbant
0,001-1% en poids d'accélérateur

et de l'agent moussant non aqueux en une quantité qui donne lieu à des densités de 0,45 à 0,6 g/cm$^3$.

**26.** Procédé de préparation des mousses de polyuréthanne selon la revendication 16, caractérisé en ce que

1. les composants (A), (B) et (C) du gel de polyuréthanne, mentionnés et définis ci-dessus en 1,
2. un matériau absorbant l'eau et
3. un agent moussant non aqueux sont combinés, mélangés les uns avec les autres et mis sous forme de mousse.

**27.** Utilisation des mousses de gels de polyuréthanne selon la revendication 16 ou des structures en feuilles préparées avec celles-ci pour la préparation de produits pour le traitement médical de blessures profondes, de blessures défectueuses et de plaies cavitaires ainsi que de produits pour la fixation de salive ou pour le séchage ou le maintien à sec d'alvéoles dentaires.

**28.** Mousses de gels de polyuréthanne selon la revendication 16, caractérisées en ce que la quantité pondérale du matériau absorbant l'eau est de 24 à 67% en poids du poids des composés polyhydroxylés.

**29.** Mousses de gels de polyuréthanne selon la revendication 16, caractérisées en ce que la quantité pondérale d'isocyanate est de 4,7-6,9% en poids du poids des composés polyhydroxylés.

**30.** Mousse de gel de polyuréthanne selon la revendication 16, caractérisée en ce que la proportion pondérale des composés polyhydroxylés est de 60-80% en poids, par rapport au poids total.

**31.** Pansements comprenant une feuille de polyuréthanne en tant que support et des mousses de gels de polyuréthanne hydrophiles appliquées sur celle-ci, pouvant être obtenues à partir de

1. un gel de polyuréthanne qui contient

(A) 25-62% en poids, de préférence 30-60% en poids, particulièrement préférablement 40-57% en poids, par rapport à la somme de (A) et (B), d'un polyuréthanne réticulé par covalence en tant que matrice à poids moléculaire élevé et
(B) 75-38% en poids, de préférence 70-40% en poids, particulièrement préférablement 60-43% en poids, par rapport à la somme de (A) et (B), d'un ou de plusieurs composés polyhydroxylés liés fermement dans la matrice par des forces de valence secondaires et ayant un poids moléculaire moyen compris entre 1 000 et 12 000, de préférence entre 1 500 et 8 000, particulièrement préférablement entre 2 000 et 6 000, et un indice d'OH moyen compris entre 20 et 112, de préférence entre 25 et 84, particulièrement préférablement entre 28 et 56, en tant qu'agent dispersant liquide, l'agent dispersant étant essentiellement exempt de composés hydroxylés ayant un poids moléculaire inférieur à 800, de préférence inférieur à 1 000, particulièrement préférablement inférieur à 1 500, et éventuellement
(C) 0-100% en poids, par rapport à la somme de (A) et (B), de charges et/ou d'additifs, et qui peut être obtenu par réaction d'un mélange de

a) un ou plusieurs polyisocyanates
b) un ou plusieurs composés polyhydroxylés ayant un poids moléculaire moyen compris entre 1 000 et 12 000, et un indice d'OH moyen compris entre 20 et 112,
c) éventuellement des catalyseurs ou des accélérateurs pour la réaction entre les groupes isocyanate et hydroxyle, et éventuellement
d) des charges et additifs connus en soi par la chimie des polyuréthannes, ce mélange étant essentiellement exempt de composés hydroxylés ayant un poids moléculaire inférieur à 800, la fonctionnalité moyenne des polyisocyanates ($F_I$) étant comprise entre 2 et 4, la fonctionnalité moyenne du composé polyhydroxylé ($Fp$) étant comprise entre 3 et 6 et l'indice d'isocyanate (K) obéissant à la

formule

$$K = \frac{300 \pm X}{(F_I \cdot F_P) - 1} + 7$$

dans laquelle X ≤ 120, de préférence X ≤ 100, particulièrement préférablement X ≤ 90 et l'indice K vaut entre 15 et 70, où les valeurs, moyennes indiquées du poids moléculaire et de l'indice d'OH doivent être comprises comme des moyennes en nombre,

    2. un matériau absorbant l'eau et
    3. un agent moussant non aqueux.

32. Pansement selon la revendication 31, caractérisé en ce que le gel de polyuréthanne contient (A) 30-60% en poids, par rapport à la somme de (A) et(B), du polyuréthanne réticulé par covalence et (B) 70-40% en poids, par rapport à la somme de (A) et (B), des composés polyhydroxylés en tant qu'agent dispersant liquide.

33. Pansement selon la revendication 31, caractérisé en ce que le gel de polyuréthanne contient

    (A) 40-57% en poids, par rapport à la somme de (A) et(B), du polyuréthanne réticulé par covalence et
    (B) 30-43% en poids, par rapport à la somme de (A) et (B), des composés polyhydroxylés en tant qu'agent dispersant liquide.

34. Pansement selon la revendication 31, caractérisé en ce que les composés polyhydroxylés sont des polyéther-polyols.

35. Pansement selon la revendication 31, caractérisé en ce que les diisocyanates sont choisis parmi le groupe constitué par les diisocyanates aliphatiques ou aromatiques non modifiés, par exemple le 4,4'-diisocyanatodiphénylméthane, et les produits modifiés formés par prépolymérisation avec des polyols ou des polyéther-polyols, par exemple le 4,4'-diisocyanato-diphénylméthane liquéfié par prépolymérisation avec du tripropylèneglycol.

36. Pansement selon la revendication 31, caractérisé en ce que les accélérateurs sont choisis parmi le groupe constitué par

- des acides organiques, en particulier l'acide p-toluènesulfonique, l'acide n-butylphosphorique
- des composés organoétain, y compris leurs sels avec des acides organiques et inorganiques, en particulier le naphténate d'étain, le benzoate d'étain, le dioctoate de dibutylétain, le diacétate de dibutylétain, l'éthylhexoate d'étain II et le diéthylhexoate de dibutylétain
- des sels de fer d'acides gras supérieurs, en particulier le stéarate de fer
- des amines, par exemple l'isophoronediamine, la méthylènedianiline, des imidazoles
- des amines tertiaires, en particulier des trialkylamines, les radicaux alkyle ayant chacun avantageusement 2-6 atomes de carbone.

37. Pansement selon la revendication 31, caractérisé en ce que le matériau absorbant l'eau est un superabsorbant.

38. Pansement selon la revendication 31, caractérisé en ce que les agents moussants non aqueux sont l'azote, l'air ou le dioxyde de carbone ou des mélanges de ces gaz.

39. Pansement selon la revendication 31, caractérisé en ce que la mousse de gel de polyuréthanne peut être obtenue à partir de

    20-95% en poids de composé polyhydroxylé
    1-30% en poids de polyisocyanate
    1-60% en poids de superabsorbant
    0,0001-10% en poids d'accélérateur

et de l'agent moussant non aqueux en une quantité qui donne lieu à des densités de 0,15 à 1,1 g/cm$^3$.

**40.** Pansement selon la revendication 31, pouvant être obtenu à partir de

55-85% en poids de composé polyhydroxylé
2-15% en poids de polyisocyanate
5-40% en poids de superabsorbant
0,001-1% en poids d'accélérateur

et de l'agent moussant non aqueux en une quantité qui donne lieu à des densités de 0,3 à 1,0 g/cm$^3$, en particulier des densités de 0,30 à 0,65 g/cm$^3$, de 0,6 à 0,9 g/cm$^3$ ou de 0,8 à 1,0 g/cm$^3$.

**41.** Procédé de préparation des pansements selon la revendication 31, caractérisé en ce que

1. les composants (A), (B) et (C) du gel de polyuréthanne, mentionnés et définis ci-dessus en 1,
2. un matériau absorbant l'eau et
3. un agent moussant non aqueux sont combinés, mélangés les uns avec les autres et mis sous forme de mousse.

**42.** Utilisation des pansements selon la revendication 31 en tant que sparadrap, emplâtre vulnéraire ou emplâtre à bulles.

**43.** Pansement selon la revendication 31, caractérisé en ce que la quantité pondérale du matériau absorbant l'eau est de 24-67% en poids, par rapport au poids du composé polyhydroxylé.

**44.** Pansements selon la revendication 31, caractérisés en ce que la densité de la mousse est de 0,3 à 0,65 g/cm$^3$, 0,6 à 0,9 g/cm$^3$ ou 0,8 à 1,0 g/cm$^3$.

**45.** Pansement selon la revendication 31, caractérisé en ce que

- l'épaisseur de la couche de gel de mousse est de 0,1-10 mm,
- la densité de la mousse est de 0,25 à 0,7 g/cm$^3$,
- la quantité d'isocyanate est de 4, 7-6, 9% en poids, par rapport au poids des composés polyhydroxylés,
- la proportion pondérale des composés polyhydroxylés est de 60-80% en poids, par rapport au poids total et
- la proportion pondérale de superabsorbant est de 20-40% en poids par rapport au poids total.

**46.** Pansement selon la revendication 31, caractérisé en ce que

- l'épaisseur de la couche de gel de mousse est de 0,05-3 mm,
- la densité de la mousse est de 0,55 à 0,9 g/cm$^3$,
- la quantité d'isocyanate est de 4,4-6,4% en poids, par rapport au poids des composés polyhydroxylés,
- la proportion pondérale des composés polyhydroxylés est de 60-80% en poids, par rapport au poids total et
- la proportion pondérale de superabsorbant est de 20-40% en poids par rapport au poids total.

**47.** Pansement selon la revendication 31, caractérisé en ce que

- l'épaisseur de la couche de gel de mousse est de 0,01-1 mm,
- la densité de la mousse est de 0,55 à 0,9 g/cm$^3$,
- la quantité d'isocyanate est de 4,4-6,4% en poids, par rapport au poids des composés polyhydroxylés,
- la proportion pondérale des composés polyhydroxylés est de 60-80% en poids, par rapport au poids total et
- la proportion pondérale de superabsorbant est de 20-40% en poids par rapport au poids total.

**48.** Pansement selon la revendication 31, caractérisé en ce qu'un côté d'une feuille de polyuréthanne est pourvu d'une couche d'une mousse de gel de polyuréthanne.